(19) Europäisches Patentamt
European Patent Office
Office européen des brevets

(11) EP 2 911 696 B1

(12) **EUROPEAN PATENT SPECIFICATION**

(45) Date of publication and mention of the grant of the patent:
**20.09.2017 Bulletin 2017/38**

(21) Application number: **13783360.4**

(22) Date of filing: **11.10.2013**

(51) Int Cl.:
***A61K 47/68*** $^{(2017.01)}$ ***A61K 31/5365*** $^{(2006.01)}$

(86) International application number:
**PCT/GB2013/052662**

(87) International publication number:
**WO 2014/064424 (01.05.2014 Gazette 2014/18)**

(54) **DRUG-PROTEIN CONJUGATES**

ARZNEIMITTELPROTEINKONJUGATE

CONJUGUÉS MÉDICAMENTS-PROTÉINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2012 US 201261717743 P**

(43) Date of publication of application:
**02.09.2015 Bulletin 2015/36**

(73) Proprietor: **Polytherics Limited**
**Babraham**
**Cambridge**
**CB22 3AT (GB)**

(72) Inventors:
- **BURT, John**
**Cambridge CD22 3AT (GB)**
- **GODWIN, Antony**
**Cambridge CD22 3AT (GB)**
- **FRIGERIO, Mark**
**Cambridge CD22 3AT (GB)**
- **BADESCU, George**
**Cambridge CD22 3AT (GB)**

(74) Representative: **Abel & Imray**
**Westpoint Building**
**James Street West**
**Bath BA1 2DA (GB)**

(56) References cited:
**WO-A1-2009/134976 WO-A1-2011/039721**

- **HANIEH KHALILI ET AL: "Comparative Binding of Disulfide-Bridged PEG-Fabs", BIOCONJUGATE CHEMISTRY, vol. 23, no. 11, 21 September 2012 (2012-09-21), pages 2262-2277, XP055096650, ISSN: 1043-1802, DOI: 10.1021/bc300372r**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 911 696 B1

## Description

This invention relates to novel drug-protein conjugates.

The specificity of binding proteins for specific markers on the surface of target cells and molecules has led to their extensive use as carriers for a variety of diagnostic and therapeutic agents. For example, such proteins conjugated to labels and reporter groups such as fluorophores, radioisotopes and enzymes find use in labelling and imaging applications, while conjugation to cytotoxic agents and chemotherapy drugs allows targeted delivery of such agents to specific tissues or structures, for example particular cell types or growth factors, minimising the impact on normal, healthy tissue and significantly reducing the side effects associated with chemotherapy treatments. Such conjugates have extensive potential therapeutic applications in several disease areas, particularly in cancer.

Water soluble, synthetic polymers, particularly polyalkylene glycols, are widely used to conjugate therapeutically active molecules such as peptide or protein ligands, including antibodies. These therapeutic conjugates have been shown to alter pharmacokinetics favourably by prolonging circulation time and decreasing clearance rates, decreasing systemic toxicity, and in several cases, displaying increased clinical efficacy. The process of covalently conjugating polyethylene glycol, PEG, to proteins is commonly known as "PEGylation".

It is important for optimised efficacy and to ensure dose to dose consistency that the number of conjugated moieties per binding protein is the same, and that each moiety is specifically conjugated to the same amino acid residue in each binding protein. Accordingly, a number of methods have been developed to improve the homogeneity of such conjugates. Liberatore et al, Bioconj. Chem 1990, 1, 36-50, and del Rosario et al, Bioconj. Chem. 1990, 1, 51-59 describe the use of reagents which may be used to cross-link across the disulfide bonds in proteins, including antibodies. WO 2005/007197 describes a process for the conjugation of polymers to proteins, using novel conjugation reagents having the ability to conjugate with both sulfur atoms derived from a disulfide bond in a protein to give novel thioether conjugates.

Maytansines are a class of cytotoxic compounds that includes maytansine itself, a natural product isolated from the east African shrub *Maytenus serrata*, and related compounds known as maytansinoids (e.g. DM1, ansamitocin P-3). Maytansine and its analogues are potent microtubule-targeted compounds that inhibit proliferation of cells at mitosis. However, due to their cytotoxicity, research has been directed to the development of conjugates containing maytansines, with a view to reducing side-effects/toxicity, improving drug delivery, or improving potency. For example, WO2009/134976 discloses antibody-drug conjugates containing hydrophilic linkers incorporating a polyethylene glycol spacer, wherein the drug may, amongst other possibilities, be a maytansinoid. WO2011/039721 discloses maytansinoids and their use to prepare conjugates with an antibody. Khalili et al., Bioconjugate Chem. 2012, 23, 2262-2277 discloses a method for conjugating PEG to an antibody fragment through a linker comprising a $-CO-Ph-CO-CH(CH_2)_2-$ moiety and capable of bis-alkylation via two sulfur atoms derived from a disulfide bond in the antibody fragment. Antibody-drug conjugates containing maytansines, such as trastuzumab emtansine (T-DM1), are currently in development for the treatment of various diseases including the treatment of cancer.

Two antibody drug conjugates have received regulatory approval: one is brentuximab vedotin, in which the drug is an auristatin, and one is trastuzumab emtansine, in which the drug is a maytansine. In both these commercially-available conjugates, the linkage of the drug to the antibody uses a linker based on maleimide. Maleimides are widely used in conjugating reagents. However, as with many other conjugating reagents, the use of maleimides presents a number of difficulties: control of the conjugation reaction is difficult, leading to products having low homogeneity, and stability of the resulting conjugates can be a problem.

Therefore, there remains a need for improved means of delivering maytansines using conjugates with good stability and homogeneity, which can be prepared effectively, and which demonstrate the required efficacy.

Accordingly, the present invention provides a maytansine-containing conjugate which has the general formula:

$$(((D_q\text{-}Lk^1)_m\text{-}P)_p\text{-}Lk^2\text{-}Lk^3)_n\text{-}Ab \qquad (I)$$

in which D represents a maytansine moiety;

q represents an integer from 1 to 10;

$Lk^1$ represents a linker;

m represents an integer from 1 to 10;

P represents a bond or a z-valent group $-P^1-NH-$ where z is from 2 to 11 and $P^1$ is a group containing at least one ethylene unit $-CH_2-CH_2-$ or ethylene glycol unit $-O-CH_2-CH_2-$;

p represents an integer from 1 to 10;

$Lk^2$ represents a bond or a y-valent linker where y is from 2 to 11 and which consists of from 1 to 9 aspartate and/or glutamate residues;

$Lk^3$ represents a linker of the general formula:

-CO-Ph-X-Y- (II)

in which Ph is an optionally substituted phenyl group; X represents a CO group or a CH.OH group; and Y represents a group of formula:

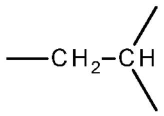
(III) or
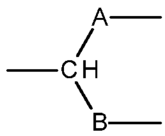
(IV)

in which each of A and B represents a $C_{1-4}$alkylene or alkenylene group;

Ab represents a binding protein or peptide capable of binding to a binding partner on a target, said binding protein being bonded to $Lk^3$ via two sulfur atoms derived from a disulfide bond in the binding protein or peptide; and

n represents an integer from 1 to s where s is the number of disulfide bonds present in the binding protein or peptide prior to conjugation to $Lk^3$;

the meanings of m, n, p, q, y and z being chosen such that the conjugate contains from 1 to 10 D groups.

D represents a maytansine moiety (i.e. the $Lk^1$ group is bonded to the residue of a maytansine). The term maytansine includes compounds such as maytansine itself, maytansinoids such as 15-methoxyansamitocin P-3, and derivatives thereof.

Preferably the maytansine is a compound containing substructure (A)

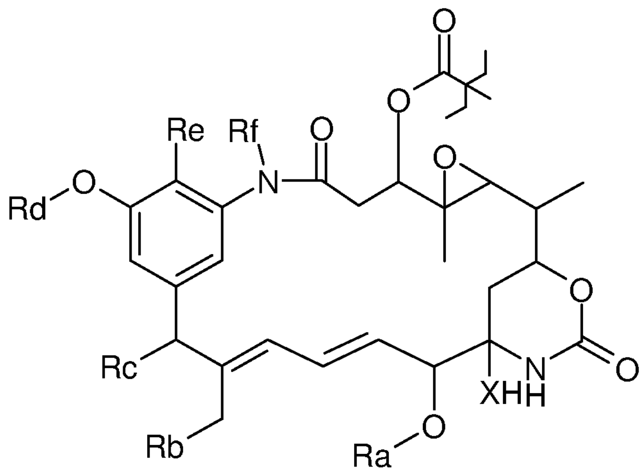
(A),

in which X represents O or S; Ra represents hydrogen or $C_{1-4}$alkyl; Rb represents hydrogen, hydroxy, $C_{1-4}$alkoxy or $C_{1-4}$alkylC(O)O-; Rc represents hydrogen, hydroxy, $C_{1-4}$alkoxy or $C_{1-4}$alkylC(O)O-; Rd represents hydrogen or $C_{1-4}$alkyl; Re represents halogen or hydrogen, and Rf represents hydrogen or $C_{1-4}$alkyl.

Preferably X represents O. Preferably Ra represents $C_{1-4}$alkyl, especially methyl. Preferably Rb represents hydrogen. Preferably Rc represents hydrogen or methoxy, more preferably hydrogen. Preferably Rd represents $C_{1-4}$alkyl, especially methyl. Preferably Re represents chlorine or hydrogen, especially chlorine. Preferably Rf represents $C_{1-4}$alkyl, especially methyl.

More preferably, the maytansine comprises substructure (B)

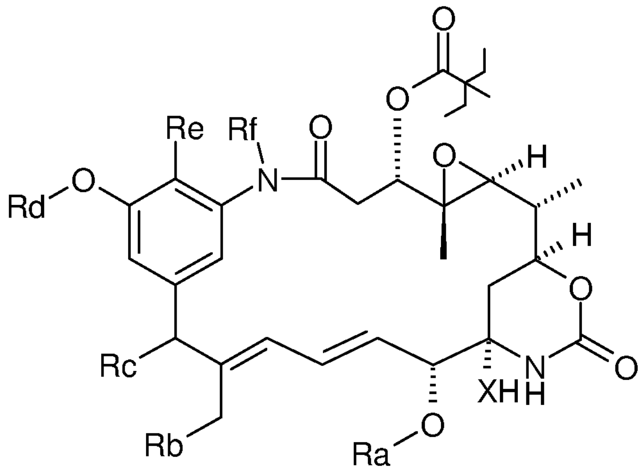

(B),

in which X and Ra-Rf have the meanings set out above.

Still more preferably, the maytansine comprises substructure (C) or (D)

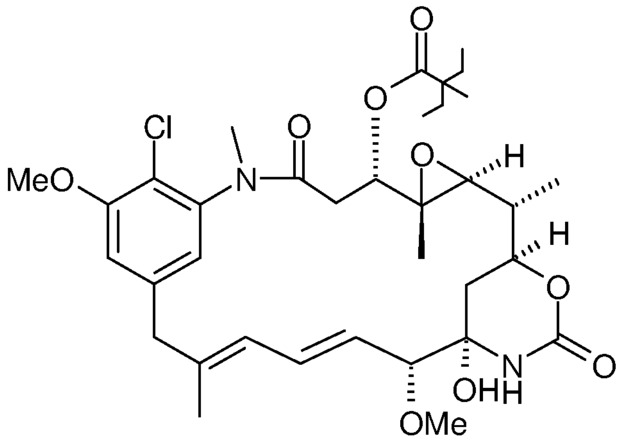

(C),

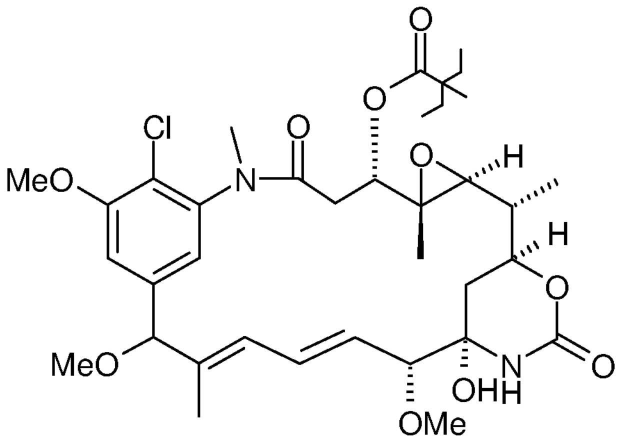

(D),

most preferably (C).

In some preferred embodiments, the maytansine includes the following group (E) bonded to the ester carbonyl carbon atom of substructure (A), (B), (C) or (D):

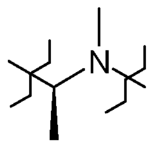
(E),

For example the maytansine may comprise substructure (F):

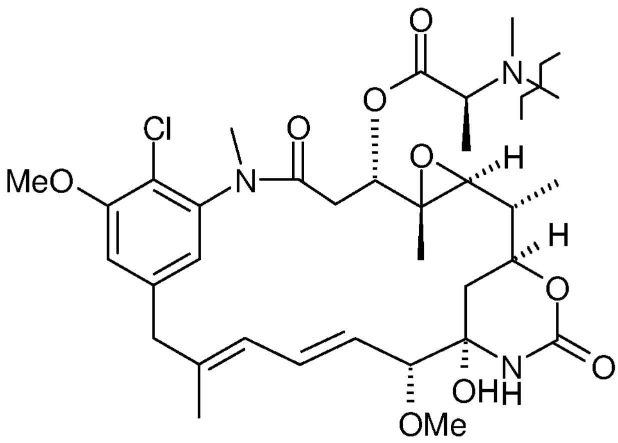
(F).

In some preferred embodiments, the maytansine includes one of the following groups bonded to the ester carbonyl carbon atom of substructure (A), (B), (C) or (D):

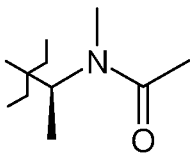
(G),
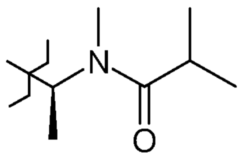
(H),
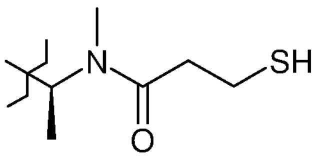
(I),

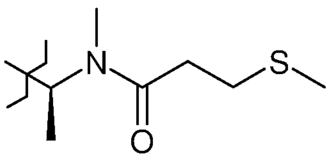
(J),
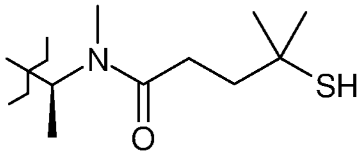
(K).

In some preferred embodiments, the maytansine contains group (L) bonded to the ester carbonyl carbon atom of substructure (A), (B), (C) or (D):

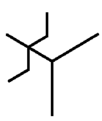
(L).

Examples of specific preferred maytansines include:

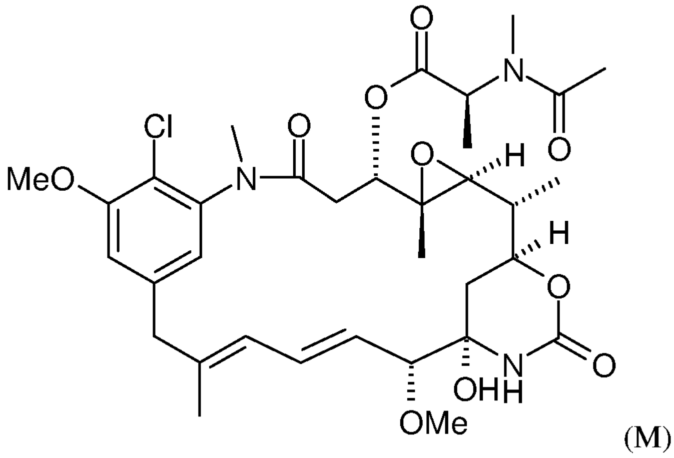

(M) (Maytansine),

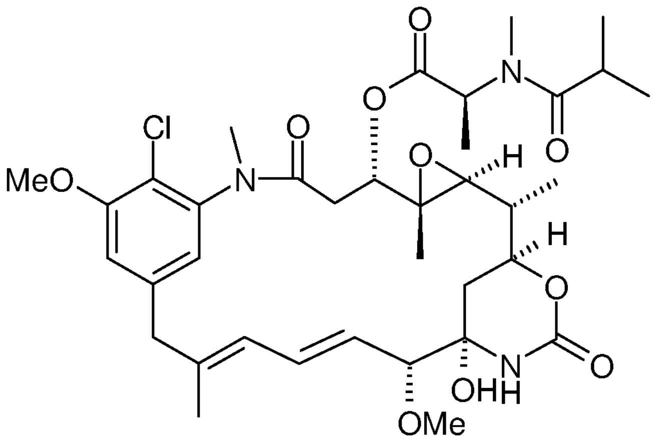

(N),

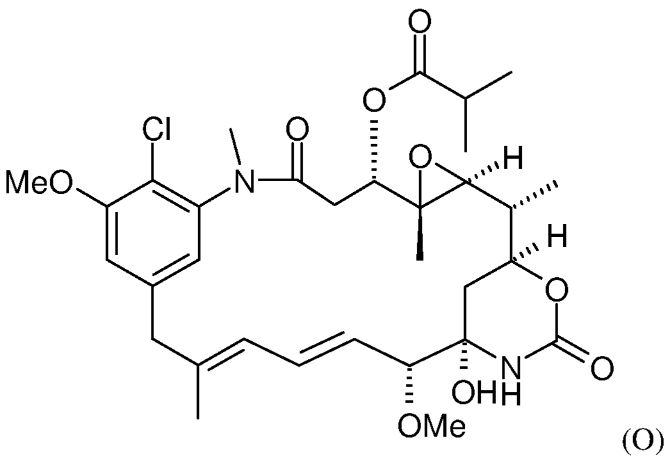

(O) (Ansamitocin P-3),

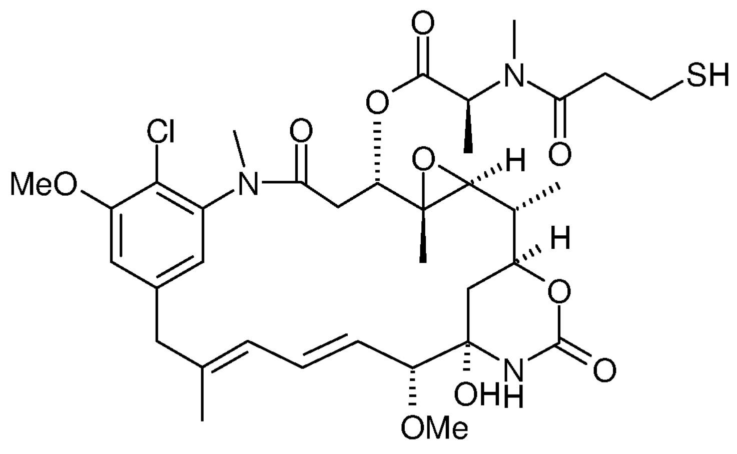

(P) (Mertansine, DM1),

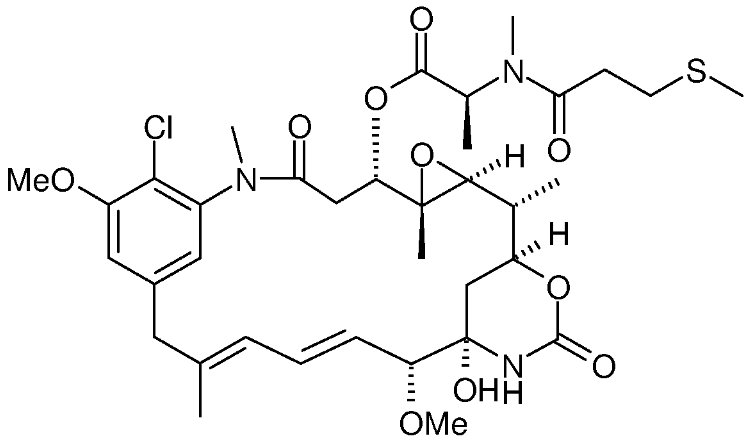

(Q) (S-methyl DM1),

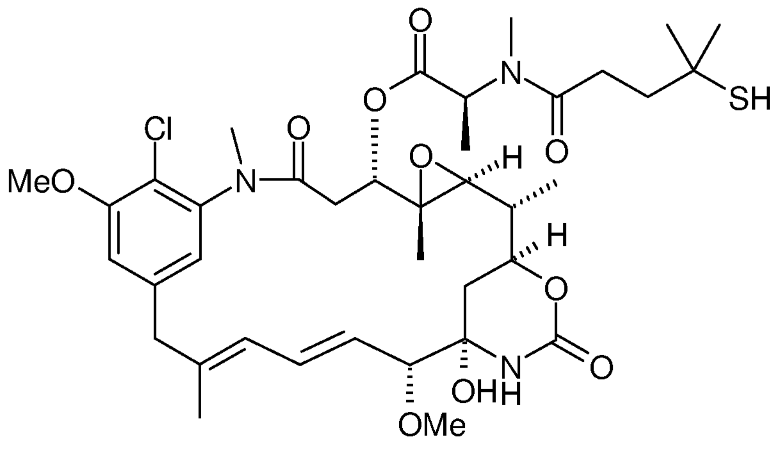

(R) (DM4),

and

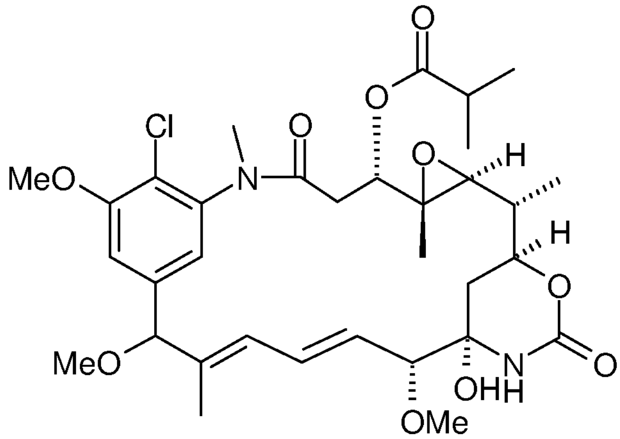

(S) (15-methoxyansamitocin P-3).

$Lk^1$ may be bonded to the maytansine moiety at any suitable point. Where the maytansine moiety corresponds to a maytansine comprising group (E), $Lk^1$ may for example be bonded to the nitrogen atom of group (E), e.g.:

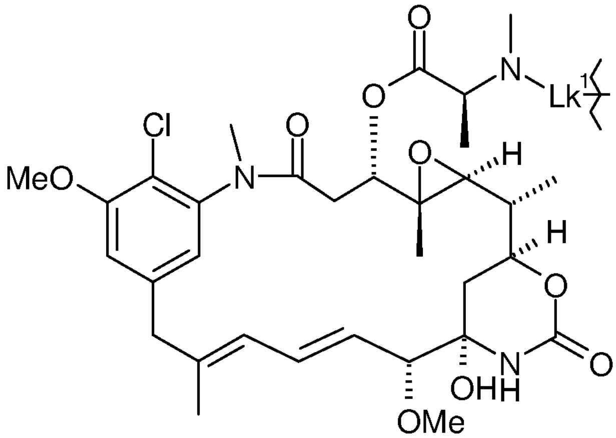

(T).

$Lk^1$ is a linker, a bond or a group which connects a maytansine moiety D to a P group. It may carry from 1 to 10 D groups. $Lk^1$ preferably contains a degradable group, i.e. $Lk^1$ is preferably a linker which breaks under physiological conditions, separating D from Ab. Alternatively, $Lk^1$ may be a linker that is not cleavable under physiological conditions. Where $Lk^1$ is a linker which breaks under physiological conditions, it is preferably cleavable under intracellular conditions. Where the target is intracellular, preferably $Lk^1$ is substantially insensitive to extracellular conditions (i.e. so that delivery to the intracellular target of a sufficient dose of the therapeutic agent is not prohibited).

Where $Lk^1$ is a degradable linker, it may contain a group that is sensitive to hydrolytic conditions. $Lk^1$ may contain a group which degrades at certain pH values (e.g. acidic conditions). Hydrolytic/acidic conditions may for example be found in endosomes or lysosomes. Examples of groups susceptible to hydrolysis under acidic conditions include hydrazones, semicarbazones, thiosemicarboazones, cis-acotinic amides, orthoesters and ketals. Examples of groups susceptible to hydrolytic conditions include:

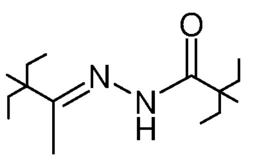

and

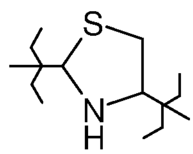

.

In a preferred embodiment, $Lk^1$ is or includes

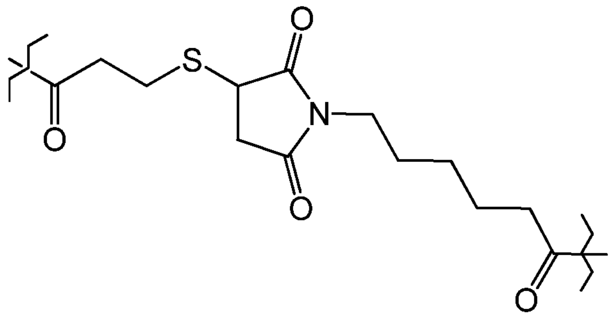

(V).

For example, $Lk^1$ may be:

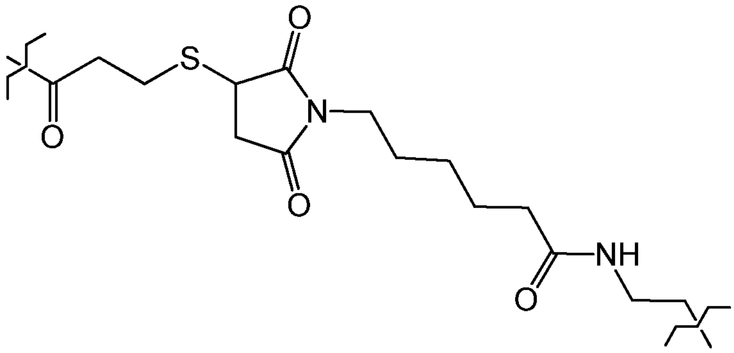

(Va),

in which case it is preferably bonded to D and P groups as shown:

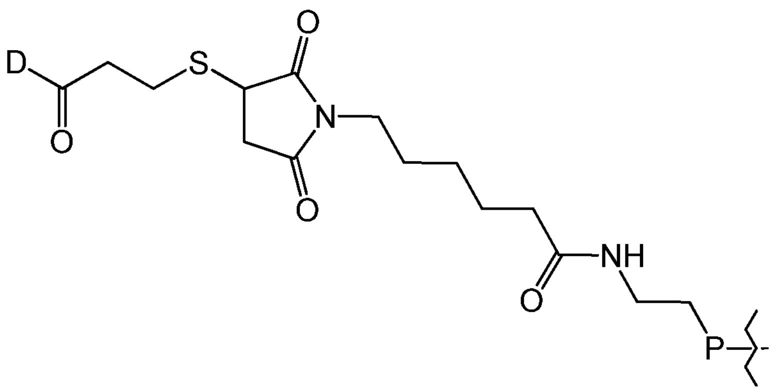

.

In that embodiment, the maytansine moiety D is preferably bonded via the nitrogen atom of group (E), e.g.:

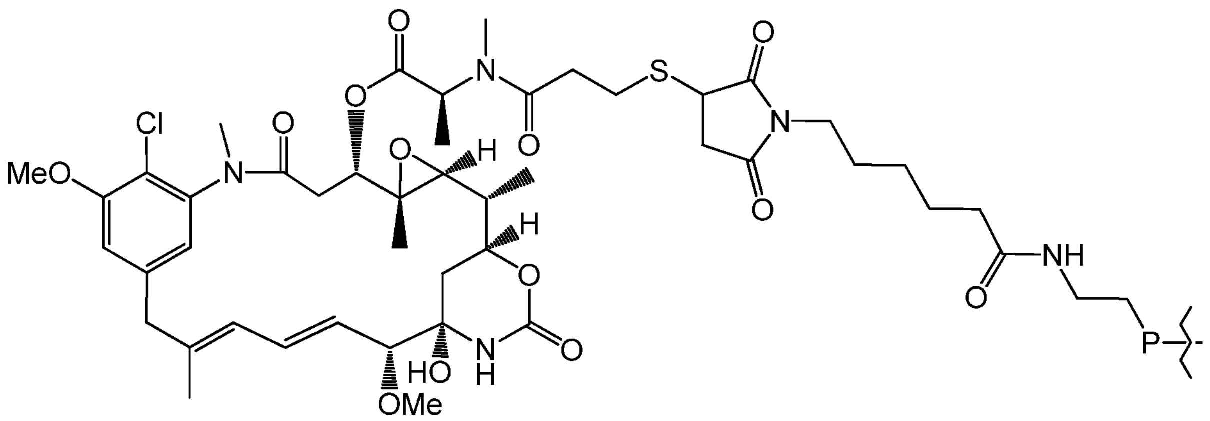

.

$Lk^1$ may also be susceptible to degradation under reducing conditions. For example, $Lk^1$ may contain a disulfide group that is cleavable on exposure to biological reducing agents, such as thiols. Examples of disulfide groups include:

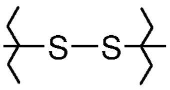
and
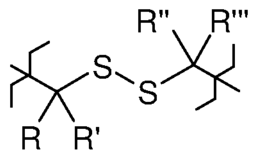

in which R, R', R" and R''' are each independently hydrogen or $C_{1-4}$alkyl. In a preferred embodiment $Lk^1$ is or includes

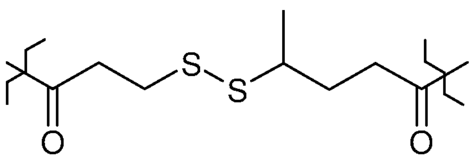
(Vb) or
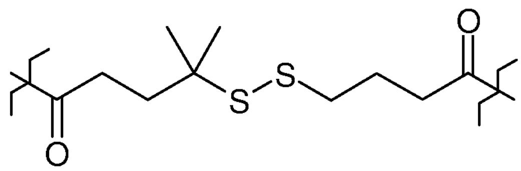
(Vc).

For example, $Lk^1$ may be

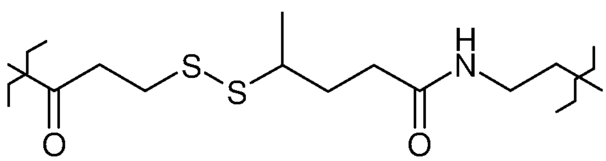
(Vd)

or

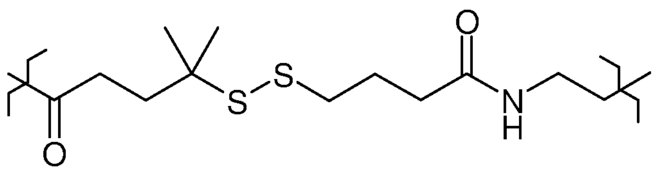
(Ve),

in which case $Lk^1$ is preferably bonded to D and P groups as shown:

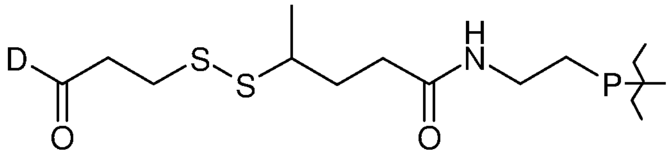

,

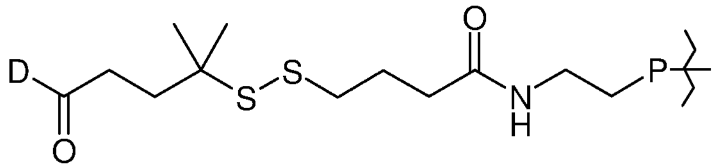

.

In those embodiments, the maytansine moiety D is preferably bonded via the nitrogen atom of group (E), e.g.

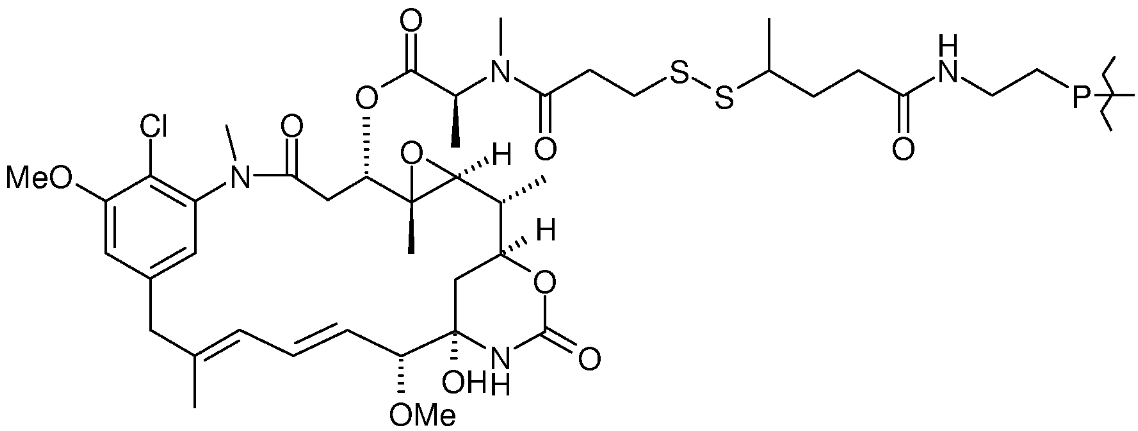

,

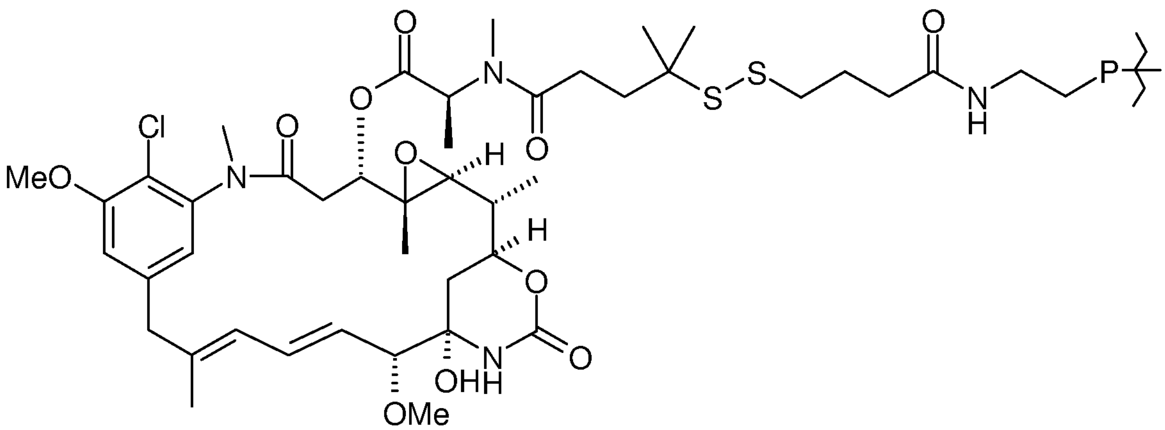

.

$Lk^1$ may also contain a group which is susceptible to enzymatic degradation, for example it may be susceptible to cleavage by a protease (e.g. a lysosomal or endosomal protease) or peptidase. For example , $Lk^1$ may contain a peptidyl group comprising at least one, for example at least two, or at least three amino acid residues (e.g. Phe-Leu, Gly-Phe-Leu-Gly, Val-Cit, Phe-Lys). For example, $Lk^1$ may be an amino acid chain having from 1 to 5, for example 2 to 4, amino acids. Another example of a group susceptible to enzymatic degradation is:

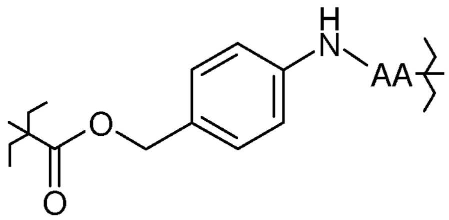

,

wherein AA represents a protease-specific amino acid sequence, such as Val-Cit.

In a preferred embodiment, $Lk^1$ is or includes:

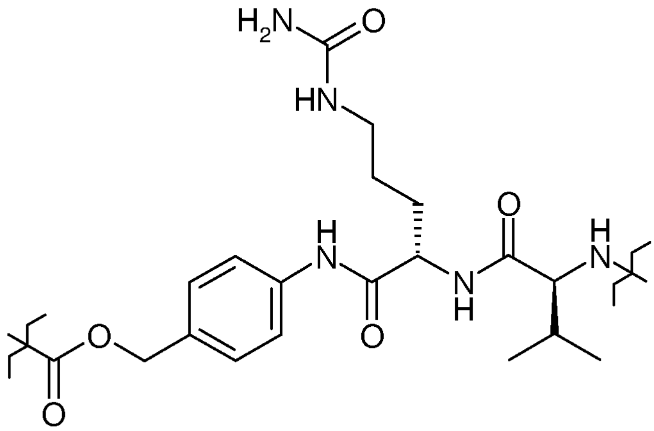

(Vf)

For example, $Lk^1$ may be

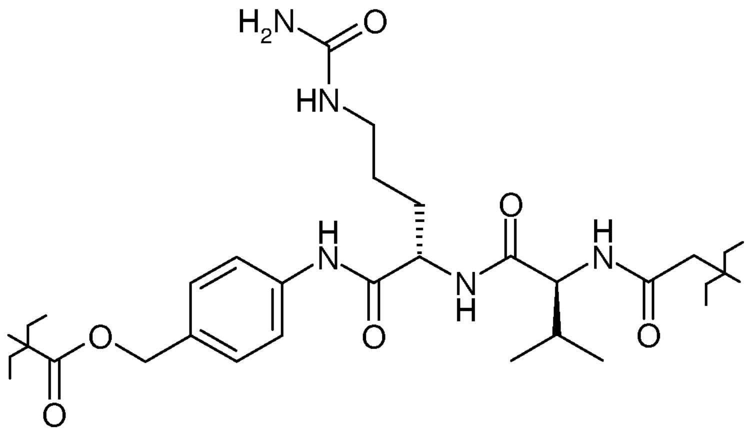

(Vg)

in which case it is preferably bonded to the D and P groups as shown below

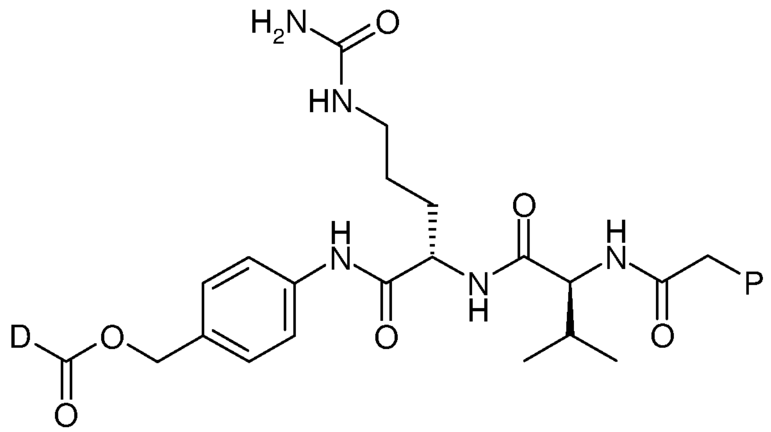

.

In that embodiment, the maytansine moiety D is preferably bonded via the nitrogen atom of group (E).

In one embodiment, $Lk^1$ carries a single maytansine moiety D (i.e. q=1). The specific linkers (Va), (Vd) and (Ve) shown above are of this type. In another embodiment, q is greater than 1, for example 2, 3 or 4, and $Lk^1$ is used as a means of incorporating more than one maytansine moiety into a conjugate of the invention. In one embodiment, this may be achieved by the use of a branching linker $Lk^1$, which may for example incorporate an aspartate or glutamate or similar residue. This introduces a branching element of formula:

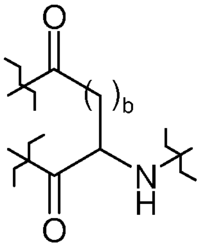

(VI)

where b is 1, 2 or 3, b=1 being aspartate and b=2 being glutamate, and b=3 representing one preferred embodiment. Each of the acyl moieties in the formula VI may be coupled to a group D via a suitable linker $Lk^{1a}$, where $Lk^{1a}$ is any suitable linker, for example a degradable linker incorporating one of the linkages mentioned above for $Lk^1$. In particular embodiments, $Lk^{1a}$ represents the group (Va), (Vd) or (Ve) shown above. The amino group of the aspartate or glutamate or similar residue may be bonded to P by any suitable means, for example the linkage may be via an amide bond, e.g. the branching group above may be connected to P via a $-CO.CH_2-$ group, thus:

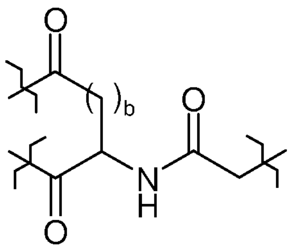

(VIa)

If desired, the aspartate or glutamate or similar residue may be coupled to further aspartate and/or glutamate and/or similar residues, for example:

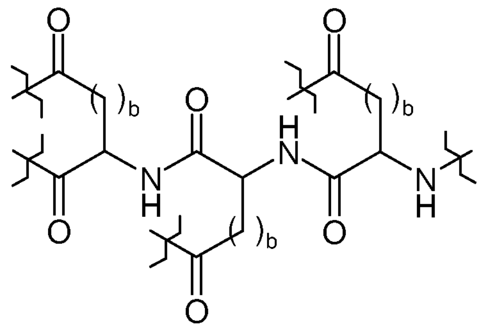

(VIIa) or

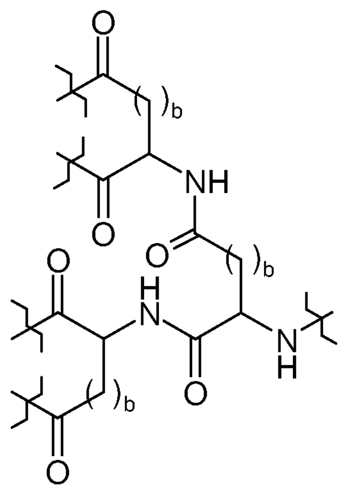

(VIIb)

and so on, up to a maximum of 9 such residues, giving the potential to incorporate up to 10 D groups. As above, each D may be attached to an aspargate/glutamate or similar residue via any suitable linker $Lk^{1a}$.

Depending on the structure of the conjugate of formula (I), it may exist in the form of a free base or free acid, in the form of a pharmaceutically acceptable salt, and/or as a solvate.

Where P represents a $-P^1$-NH- group, $P^1$ contains at least one ethylene or ethylene glycol unit ($-CH_2-CH_2-$ or $-O-CH_2-CH_2-$). If many such units are present, $P^1$ represents polyethylene, PE, or polyethylene glycol, PEG. These polymers may contain a single linear chain, or may have branched morphology composed of many chains either small or large, in which case they will contain branching groups, typically containing >CH-, as for example in $-(CH_2CH_2)_2-CH-$ or $-(O-CH_2-)_2CH-$. They may optionally be derivatised or functionalised in any desired way. They may for example carry an additional therapeutic agent, or a labelling agent. Multimeric conjugates that contain more than one molecule of therapeutic agent, for example more than one molecule of an maytansine, or a molecule of a therapeutic agent in addition to a molecule of a maytansine, can result in synergistic and additive benefits. Where $P^1$ represents PE or PEG, the optimum molecular weight of the polymer will of course depend upon the intended application. Generally, where $P^1$ represents PE, it is preferred that the number average molecular weight is up to 2kDa, preferably up to 1kDa. Where $P^1$ represents PEG, higher molecular weights may be used, for example the number average molecular weight may be up to 75kDa, for example up to 60kDa, with the minimum number average molecular weight being for example at least 0.5kDa, for example at least 1kDa, for example 2kDa. In one preferred embodiment, PEG of number average molecular weight of from 0.5 to 2kDa may be used. However, in some preferred embodiments, P may be a bond, or P may represent $-P^1$-NH- wherein $P^1$ contains a small number of discrete ethylene or ethylene glycol units, for example from 2 to 10, for example 2 or 3, ethylene or, preferably, ethylene glycol units.

If it is desired for the conjugate of formula I to contain more than one $-(CH_2-CH_2)_a-$ or $-(O-CH_2-CH_2)_a-$ chain (where a is the number of ethylene or ethylene glycol units in any linear chain), for example so that each such chain may carry a $D_q-Lk^1$ group, this may be achieved either by bonding more than one (i.e. from 2 to 10) such chains to $Lk^2$, or by using a branched PE or PEG, in which case only one group P will be attached to $Lk^2$, but this will contain more than one branch, for example from 1 to 9 branches (providing from 2 to 10 attachment points for $D-Lk^1$ groups).

It will be understood that where P is $-P^1$-NH-, the or each P group is coupled to adjacent groups $Lk^1$ and/or $Lk^2$ via an amide bond. For example PEG (which normally terminates with an -OH group) may be converted into the corresponding PEG amine, which terminates with an $-NH_2$ group, for amide bond formation with a $-CO_2$ group in, say, $Lk^2$; or the OH group may be reacted to form a linkage $-NH.CO.CH_2.O-$ with $Lk^1$ as described above.

In a preferred embodiment of the invention, $P^1$ represents PEG, a water-soluble, synthetic polymer, and throughout this specification, except where the context requires otherwise, any general reference to $P^1$ should be understood to include a specific reference to PEG.

$Lk^2$ represents a y-valent linker where y is from 2 to 11. It is thus capable of bonding from 1 to 10 groups P or $Lk^1$. In its simplest form, $Lk^2$ is a bond, in which case $Lk^1$ is bonded directly to a $-P^1$-NH- group or, if P is a bond, to a $D-Lk^1$ group. However, $Lk^2$ may be used as a means of incorporating more than one D group (maytansine moiety) into the conjugates of the invention. This is achieved by coupling an aspartate or glutamate residue to the -CO-group of $Lk^3$ via an amide linkage (e.g. reacting the aspartate or glutamate amine group with a suitable carboxylic acid derivative corresponding to $Lk^3$). This introduces a branching element of formula VI shown above. In that case, each of the acyl moieties may be coupled to a $-P^1$-NH- group via an amide linkage, or when P is a bond, to a $D-Lk^1$ group. Alternatively the aspartate or glutamate residue may be coupled to further aspartate and/or glutamate residues, as shown in formulae

VIIa and VIIb shown above, and so on, up to a maximum of 9 such residues, giving the potential to incorporate up to 10 D groups via bonding of multiple D-$Lk^1$-P groups at different attachment points in $Lk^2$. It will be understood that the valency of $Lk^2$ is associated with the number of D-$Lk^1$-P groups present. For example, when P is -$P^1$-NH-, for each D-$Lk^1$-P group, the valency of $Lk^2$ is associated with the number of -$P^1$-NH- groups present, i.e. p will equal y-1. When P is a bond, for each D-$Lk^1$-P group, the valency of $Lk^2$ is associated with the number of groups D-$Lk^1$ present, i.e. m will equal y-1.

$Lk^3$ is a specific linker capable of binding to a binding protein via two sulfur groups derived from a disulfide bond in the binding protein. In $Lk^3$, the phenyl group Ph may be unsubstituted or substituted. Substituents which may optionally be present on the phenyl group Ph include for example one or more of the same or different substituents selected from alkyl (preferably $C_{1-4}$alkyl, especially methyl, optionally substituted by OH or $CO_2H$), -CN, - $NO_2$, -$CO_2R^4$, -COH, -$CH_2OH$, -$COR^4$, -$OR^4$, -$OCOR^4$, -$OCO_2R^4$, -$SR^4$, -$SOR^4$, -$SO_2R^4$,-$NHCOR^4$, -$NR^4COR^4$, $NHCO_2R^4$, -$NR^4.CO_2R^4$, -NO, -NHOH, -$NR^4$.OH, -C=N-$NHCOR^4$, -C=N-$NR^4.COR^4$, -$N^+R^4{}_3$, -$N^+H_3$, -$N^+HR^4{}_2$, -$N^+H_2R^4$, halogen, for example fluorine or chlorine, -C=$CR^4$, -C=$CR^4{}_2$ and -C=$CHR^4$, in which each $R^4$ independently represents a hydrogen atom or an alkyl (preferably $C_{1-6}$alkyl), aryl (preferably phenyl), or alkyl-aryl (preferably $C_{1-6}$alkyl-phenyl) group. The presence of electron withdrawing substituents is especially preferred. Preferred substituents include for example -CN, -$NO_2$, -$OR^4$, -$OCOR^4$, -$SR^4$, -$NHCOR^4$, -NR.$COR^4$, -NHOH and -$NR^4.COR^4$. Preferably, however, the phenyl group Ph is unsubstituted.

When Y represents the group III, a single carbon bridge is formed between the linker $Lk^3$ and two sulfur atoms derived from a disulfide bond in the binding protein Ab, and when Y represents the group IV, the nature of the groups A and B determine the length of the bridge which is formed between the linker $Lk^3$ and two sulfur atoms derived from a disulfide bond in the binding protein Ab. Preferably, a 3-carbon bridge is formed, i.e. preferably Y has the formula:

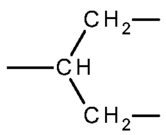

(IVa)

As mentioned above, conjugates which contain more than one maytansine moiety may have advantages. The presence of more than one maytansine moiety may be achieved in a number of different ways, for example as described above by the use of a branched PEG, by the use of a multivalent group $Lk^2$, or by the use of a multivalent group $Lk^1$. It may however also be achieved by attaching more than one linker $Lk^3$ to the binding protein Ab. In general, normal full-length antibodies have 4 interchain disulfide bonds (heavy-heavy chain or heavy-light chain for whole IgG1 antibodies), and any or all of these can be bridged by the linker $Lk^3$ according to the invention. It is also envisaged that one or more intrachain disulfide bonds in the antibody may be bridged by a linker $Lk^3$. Where more than one conjugating group is present, n is greater than 1. n may for example be 2, 3, or 4.

Alternatively or in addition, one or more additional maytansine moieties can be present linked via a linker $Lk^1$ to P or, where P is a bond, directly to $Lk^2$. In this case, m is greater than 1, for example 2, 3 or 4, up to a maximum of 10. If more than one linker $Lk^1$ is present, these may be the same as each other, or different.

Alternatively or in addition, one or more additional maytansine moieties can be present linked to a multivalent linker $Lk^1$. In this case, q is greater than 1, for example 2, 3 or 4, up to a maximum of 10.

It is envisaged that conjugates of the present invention may carry up to 10 D groups (maytansine moieties). Where it is desired for the conjugate of formula I to contain more than one D group (i.e. more than one maytansine moiety), this may be achieved in any one of a number of ways. For example, multiple $(((D_q\text{-}Lk^1)_m\text{-}P)_p\text{-}Lk^2\text{-}Lk^3)$- groups may be bonded to a single antibody (i.e. n is from 2 to s). This mode of attachment forms one preferred way of providing conjugates containing more than one group D. A second preferred way of providing conjugates containing more than one group D is by use of a multivalent linker $Lk^1$, for example a linker $Lk^1$ which contains one or more aspartate and/or glutamate or similar residues as described above (as for example in formulae VI, VIIa and VIIb), allowing multiple D groups to be present (i.e. q is from 2 to 10). It is believed that conjugates in which $Lk^1$ is a multivalent linker, especially one including formula VI above, p=1, q=2, m=1 and $Lk^2$ is a bond, are particularly effective, and such conjugates form a preferred embodiment of the invention.

Alternatively or in addition, where $Lk^2$ is a group consisting of from 1 to 9 aspartate and/or glutamate residues, multiple $((D_q\text{-}Lk^1)_m\text{-}P)$- groups may be bonded at different positions on the $Lk^2$ group (i.e. p is from 2 to 10), by amide bonding of each group through an amine moiety with a carboxyl group of an aspartate or glutamate residue in the $Lk^2$ group. Where $P^1$ contains at least one ethylene or ethylene glycol unit and also contains at least one branching unit, multiple $(D_q\text{-}Lk^1)$- groups may additionally or alternatively be bonded at different positions on the $P^1$ group (i.e. m is from 2 to 10).

Conjugates having combinations of the above are also disclosed. By way of example, a conjugate may contain

an Ab group bonded to two $((D\text{-}Lk^1)_m\text{-}P)_p\text{-}Lk^2\text{-}Lk^3$- groups in which, for each of those $((D\text{-}Lk^1)_m\text{-}P)_p\text{-}Lk^2\text{-}Lk^3$- groups, $Lk^2$ is an aspartate or glutamate residue bonded to two $(D\text{-}Lk^1)_m$-P groups, and in which, for each of those $(D\text{-}Lk^1)_m$-P groups, P is -$P^1$-NH- in which $P^1$ contains at least one ethylene or ethylene glycol unit and also contains at least one branching unit, so that in total 8 D-$Lk^1$ groups are present in the conjugate. By way of further example, a conjugate may contain an Ab group bonded to two $((D\text{-}Lk^1)_m\text{-}P)_p\text{-}Lk^2\text{-}Lk^3$- groups in which, for each of those $((D\text{-}Lk^1)_m\text{-}P)_p\text{-}Lk^2\text{-}Lk^3$- groups, $Lk^2$ is a bond, P is -$P^1$-NH- in which $P^1$ contains at least one ethylene or ethylene glycol unit and also contains at least one branching unit, and each $Lk^1$ contains an aspartate or glutamate residue bonded to two D groups, so that in total 8 D-$Lk^1$ groups are present in the conjugate.

Different $Lk^3$, $Lk^2$, P, $Lk^1$ and D groups may also be present in the same conjugate, for example where a conjugate contains an Ab group bonded to two $((D\text{-}Lk^1)_m\text{-}P)_p\text{-}Lk^2\text{-}Lk^3$ groups, in one of those $((D\text{-}Lk^1)_m\text{-}P)_p\text{-}Lk^2\text{-}Lk^3$- groups $Lk^2$ may be a bond and in the other of those $((D\text{-}Lk^1)_m\text{-}P)_p\text{-}Lk^2\text{-}Lk^3$- groups $Lk^2$ may be an aspartate or glutamate residue. Similarly, where a conjugate contains multiple $(D\text{-}Lk^1)_m$-P groups bonded to an $Lk^2$ group, for one of those groups P may be a bond, and for another of those groups P may be -$P^1$-NH-. In its simplest embodiment, the invention relates to conjugates which contain a single maytansine moiety (n=m=q=p=1). The conjugates may contain up to 10 maytansine moieties, for example up to 8 maytansine moieties. They may for example contain up to 4, for example 1, 2, 3 or 4, maytansine moieties. Where two D groups are present, these may for example be in conjugates of the formulae:

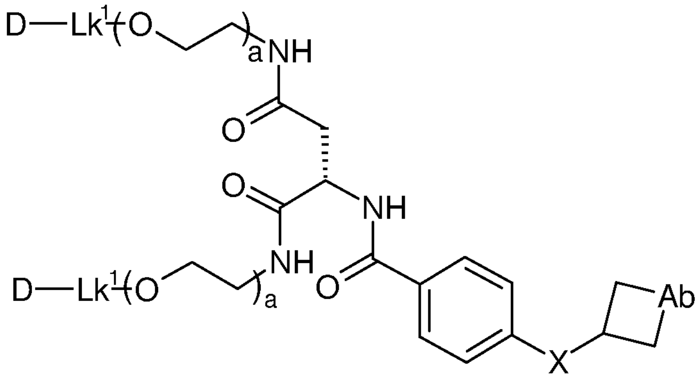

(Ia),

or

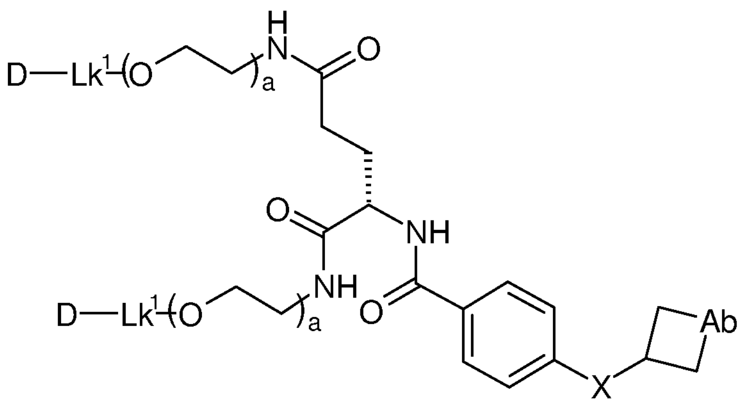

(Ib),

in which $Lk^1$ preferably comprises a group of formula (Va), (Vd) or (Ve) as described above.

Alternatively, where two D groups are present, these may for example be in conjugates of the formulae:

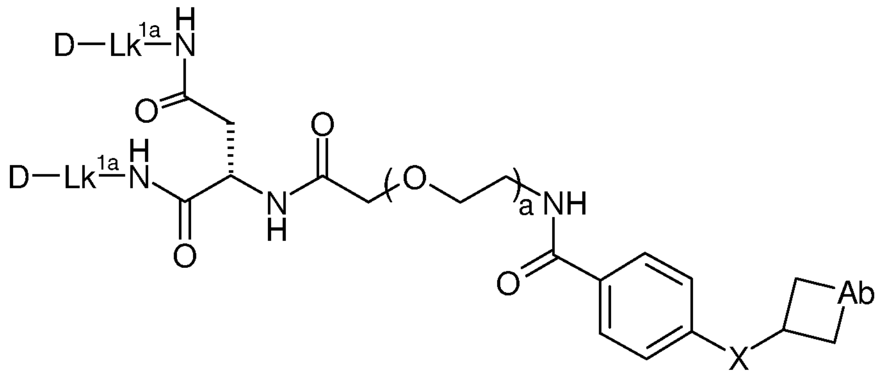

(Ic),

or

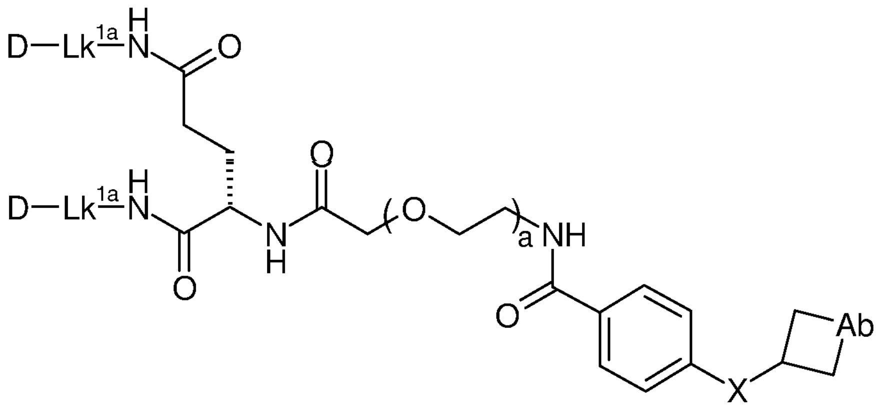

(Id).

The above formulae show the bonding of X across one of the disulfide bonds present in Ab. Antibodies may contain up to 4 inter-chain disulfide bonds, and if each of these bonds is bridged by a reagent carrying a single maytansine molecule, the resulting conjugate will have a drug:antibody ratio (DAR) of 4. If a reagent carrying two maytansine molecules is used to bridge all 4 disulfide bonds, for example a reagent carrying two PE or PEG chains or having a branched PE or PEG chain or having a branched linker $Lk^1$, then the DAR will be 8. Conjugates having such high DARs may have significant clinical advantages. Conjugates of the above formulae Ia-Ie above but in which Ab carries 2, 3 or, especially, 4, copies of the ~X- group, form one preferred embodiment of the invention.

For convenience, the term "binding protein" is used throughout this specification to include both binding proteins and peptides, and except where the context specifically requires otherwise, should be understood to include peptides as well as proteins. Binding proteins that can be used in the conjugates of the invention include any protein, polypeptide or peptide that can serve as a binding agent for a binding partner on a target. The target may be for example a micro-organism, a virus, or a cell, for example a cancer or immune cell. The binding protein thus acts to target the maytansine to the particular target. Examples of such binding proteins include full length antibodies, antibody fragments, immunoglobulin (Ig) and non-Ig protein scaffolds obtained by rational or combinatorial protein engineering techniques, and lectins. The most common binding proteins used in protein-drug conjugates are antibodies, and any reference to a binding protein or to the group Ab should, except where the context specifically requires otherwise, be understood to include a specific reference to an antibody. Throughout this specification, the term "antibody" should be understood to mean an immunoglobulin molecule that recognises and specifically binds to a target antigen, such as a protein, polypeptide, peptide, carbohydrate, polynucleotide, lipid, or combination thereof through at least one antigen recognition site within the variable region of the immunoglobulin molecule. The term "antibody" encompasses polyclonal antibodies, monoclonal antibodies, multispecific antibodies such as bispecific antibodies, chimeric antibodies, humanised antibodies, human antibodies, fusion proteins comprising an antigen determination portion of an antibody, and any other modified immunoglobulin molecule comprising an antigen recognition site so long as the antibodies exhibit the desired biological activity. An antibody can be of any the five major classes of immunoglobulins: IgA, IgD, IgE, IgG, and IgM, or subclasses (isotypes) thereof (e.g. IgG1, IgG2, IgG3, IgG4, IgAl and IgA2), based on the identity of their heavy-chain constant domains referred to as alpha, delta, epsilon, gamma, and mu, respectively. The different classes of immunoglobulins have different and well known subunit structures and three-dimensional configurations. The use of IgG1 or IgG4 is

particularly preferred.

Further, except where the context requires otherwise, the term "antibody" should be understood to encompass full length antibodies and antibody fragments comprising an antigen-binding region of the full length antibody. Antibody fragments may for example be Fab, Fab', $F(ab')_2$, scFv, Fv, diabodies, minibodies or multispecific antibodies formed from antibody fragments, for example minibodies composed of different permutations of scFv fragments or diabodies, and optionally Fc fragments or $C_H$ domains, such as scFv-Fc, scFv-Fc-scFv, Fab-scFv, $(Fab'ScFv)_2$, scDiabodies, scDiabody-Fc, scDiabody-$C_H3$, scFv- $C_H3$, scFv-$C_H2$-$C_H3$ fusion proteins and so forth. An antibody fragment can be produced by enzymatic cleavage, synthetic or recombinant techniques.

A binding protein can serve as a binding agent for a receptor, antigen or other moiety on the surface of a target, for example a cell or virus associated with a proliferative, autoimmune or infectious disease. For example, the binding protein may be an antibody that specifically binds to a cell surface antigen on a cancer cell. Methods of identification and validation of cell surface antigens for antibody targeting of cancer cells are known, for example in Carter P, et al., Endocr. Relat. Cancer. 2004 Dec;11(4):659-87, and a number of antibody-drug conjugates for treating cancer are currently in clinical development. Examples of antibodies available for the treatment of cancer, and tumour markers of specific cancers, are also well known in the art and can be used. Alternatively, the target may be an immune cell, for example a cell that is responsible for producing autoimmune antibodies, or an activated lymphocyte that is associated with an autoimmune disease. In other embodiments, the target may be a micro-organism or virus associated with a microbial or viral infection or disease.

Conjugates of the present invention may be prepared by reducing one or more disulfide bonds in a binding protein and subsequently reacting with a conjugating reagent of the general formula:

$$((D_q\text{-}Lk^1)_m\text{-}P)_p\text{-}Lk^2\text{-}Lk^{3a} \qquad \text{(VIII)}$$

in which D, $Lk^1$, P, $Lk^2$ and m, p and q have the meanings given for the general formula I, and $Lk^{3a}$ represents a group of formula:

$$\text{-CO-Ph-}X^a\text{-}Y^a \qquad \text{(IX)}$$

in which Ph has the meaning given above, $X^a$ represents a CO group, and $Y^a$ represents a group:

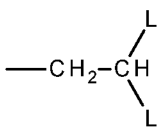
(X),
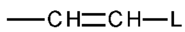
(XI),
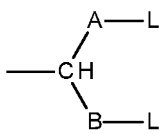
(XII)

or

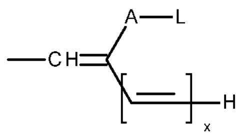
(XIII)

in which A and B have the meanings given above, each L independently represents a leaving group, and x represents an integer from 1 to 4.

Groups of formulae X, XI, XII and XIII above are chemical equivalents of each other, with groups of formula X and XI leading to a single carbon bridge across a disulfide bond of the antibody, and groups of formula XII and XIII leading to longer carbon bridges, shown below for the case where n=1:

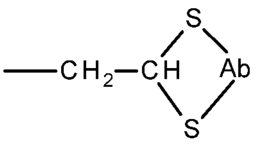

(XIV) or

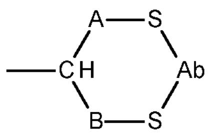

(XV)

When reacting a conjugating reagent containing a group X or XII with an antibody, the immediate step in the reaction pathway is loss of one leaving group L leading to a conjugating reagent containing a group XI or XIII, respectively. Thus, conjugating reagents of formula XI or XIII are either prepared *in situ*, or are used *ab initio.* A key feature of using conjugation reagents containing any of groups X, XI, XII or XIII, is that an $\alpha$-methylene leaving group and a double bond are cross-conjugated with an electron withdrawing function that serves as a Michael activating moiety. If the leaving group is prone to elimination in the cross-functional reagent rather than to direct displacement and the electron-withdrawing group is a suitable activating moiety for the Michael reaction then sequential intramolecular bis-alkylation can occur by consecutive Michael and retro Michael reactions. The leaving moiety serves to mask a latent conjugated double bond that is not exposed until after the first alkylation has occurred and bis-alkylation results from sequential and interactive Michael and retro-Michael reactions. The electron withdrawing group and the leaving group are optimally selected so bis-alkylation can occur by sequential Michael and retro-Michael reactions. It is also possible to prepare cross-functional alkylating agents with additional multiple bonds conjugated to the double bond or between the leaving group and the electron withdrawing group.

A leaving group L may for example represent $-SR^4$, $-SO_2R^4$, $-OSO_2R^4$, $-N^+R^4_3$, $-N^+HR^4_2$, $-N^+H_2R^4$, halogen, or -OØ, in which $R^4$ has the meaning given above, and Ø represents a substituted aryl, especially phenyl, group, containing at least one electron withdrawing substituent, for example -CN,$-NO_2$, $-CO_2R^4$, -COH, $-CH_2OH$, $-COR^4$, $-OR^4$, $-OCOR^4$, $-OCO_2R^4$, $-SR^4$,$-SOR^4$, $-SO_2R^4$, $-NHCOR^4$, $-NR^4COR^4$, $-NHCO_2R^4$, $-NR^4CO_2R^4$, -NO, -NHOH, $-NR^4OH$, $-C{=}N\text{-}NHCOR^4$, $-C{=}N\text{-}NR^4COR^4$, $-N^+R^4_3$, $-N^+HR^4_2$, $-N^+H_2R^4$, halogen, especially chlorine or, especially, fluorine, $-C{\equiv}CR^4$, $-C{=}CR^4_2$ and $-C{=}CHR^4$, in which each $R^4$ independently has one of the meanings given above. An especially preferred leaving group L is $-SR^4$ or $-SO_2R^4$, especially $-SO_2R^4$, where $R^4$ represents a phenyl or, especially, a tosyl group. Thus, a particularly preferred group $Y^a$ is:

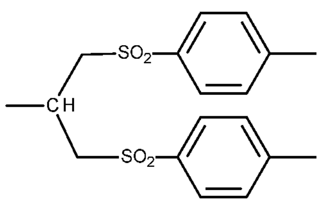

(XIIa)

Examples of preferred conjugating reagents include:

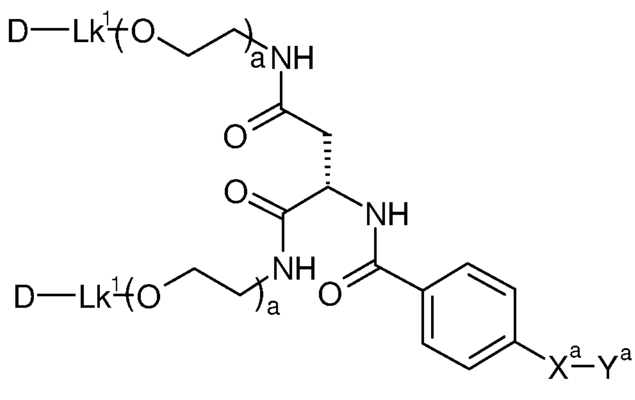

(VIIIa),

and

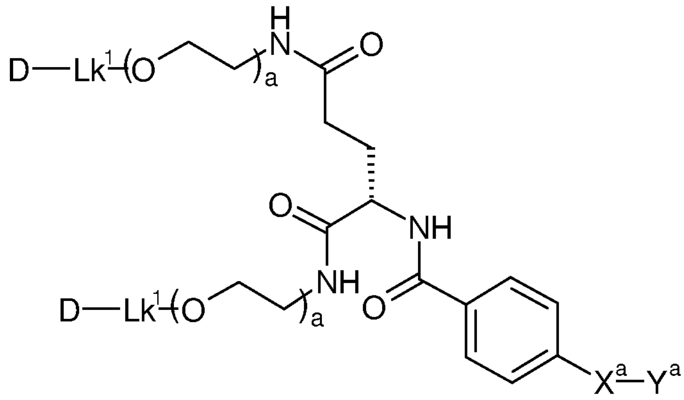

(VIIIb),

in which $Lk^1$ preferably comprises a group of formula (Va), (Vd) or (Ve) as described above, and in which $Y^a$ is preferably a group of formula (XII), especially in which A and B are each $-CH_2-$.

Further preferred examples of conjugating agents include:

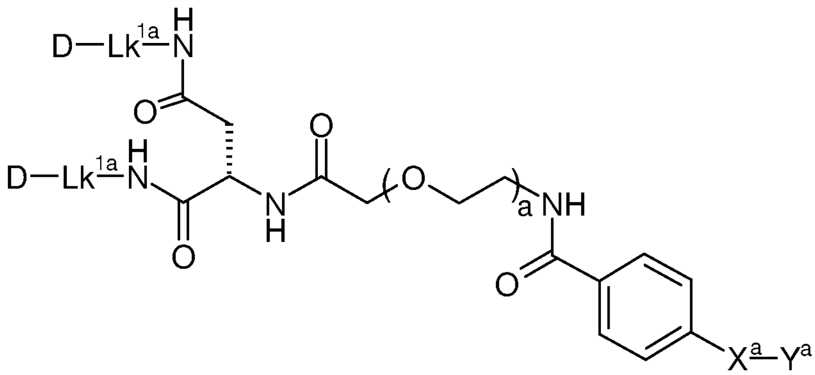

(VIIIc),

and

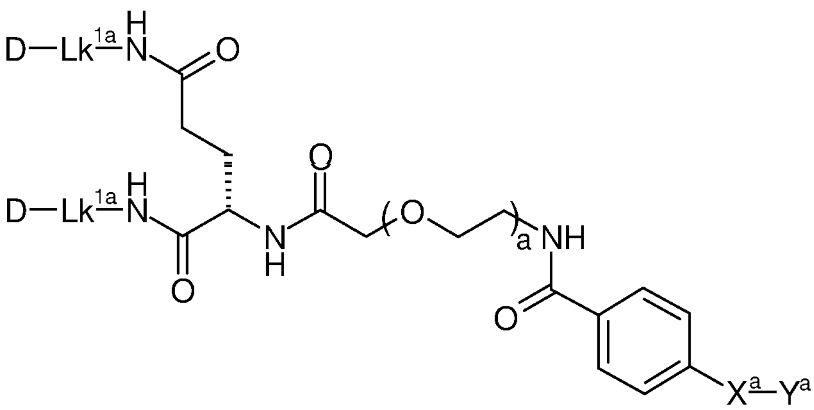

(VIIId),

in which $Y^a$ is preferably a group of formula (XII), especially in which A and B are each $-CH_2-$.

The immediate product of the conjugation process using one of the reagents described above is a maytansine-antibody conjugate in which X represents a keto group CO. However, the process of the invention is reversible under suitable conditions. This may be desirable for some applications, for example where rapid separation of the maytansine from the antibody is required, but for other applications, rapid separation may be undesirable. It may therefore be desirable to stabilise the conjugates by reduction of the CO group X to give a CH.OH group X. Accordingly, the process described above may comprise an additional optional step of reducing the initially-formed CO group X in $Lk^3$ to give a conjugate having a CH.OH group X in $Lk^3$. The use of a borohydride, for example sodium borohydride, sodium cyanoborohydride,

potassium borohydride or sodium triacetoxyborohydride, as reducing agent is particularly preferred. Other reducing agents which may be used include for example tin(II) chloride, alkoxides such as aluminium alkoxide, and lithium aluminium hydride.

Suitable reaction conditions for the process described above are given in WO 2005/007197 and WO 2010/100430. The process may for example be carried out in a solvent or solvent mixture in which all reactants are soluble. The antibody may be allowed to react directly with the conjugation reagent in an aqueous reaction medium. This reaction medium may also be buffered, depending on the pH requirements of the nucleophile. The optimum pH for the reaction will generally be at least 4.5, typically between about 5.0 and about 8.5, preferably about 6.0 to 8.2. The optimal reaction conditions will of course depend upon the specific reactants employed.

Reaction temperatures between 3-37°C are generally suitable. Reactions conducted in organic media (for example THF, ethyl acetate, acetone) are typically conducted at temperatures up to ambient.

The binding protein can be effectively conjugated with the desired reagent using a stoichiometric equivalent or an excess of reagent. Excess reagent and the product can be easily separated during routine purification, for example by standard chromatography methods, e.g. ion exchange chromatography or size exclusion chromatography, diafiltration, or, when a polyhistidine tag is present, by separation using metal affinity chromatography, e.g. based on nickel or zinc. Targeting of specific disulfide bonds in the binding protein may be carried out by known methods; for example, by partial reduction of the protein, see for example Liu et al, Anal. Chem. 2010, 82, 5219-5226.

Conjugation reagents of the general formulae VIII above may be prepared by reacting a maytansine with a compound of the general formula:

$$((Lk^{1b})_m\text{-}P)_p\text{-}Lk^2\text{-}Lk^{3a}$$

in which m, P, L, p, $Lk^2$ and $Lk^{3a}$ have the meanings given for the general formula VIII and $Lk^{1b}$ is a group of formula $Lk^1$ modified to include a group reactive with a group present in a maytansine. Typical groups and suitable reactions are well known to the skilled man. Conjugation reagents of the general formulae VIII are novel, and the invention therefore provides these reagents *per se*. In these novel reagents, the preferred meanings for $Lk^1$, P, $Lk^2$, m, p and q are as given above for the general formula I. The invention further provides a pharmaceutical composition comprising a maytansine-protein conjugate according to the invention, together with a pharmaceutically acceptable carrier, optionally together with an additional therapeutic agent; such a conjugate for use in therapy, specifically, for use as a medicament for the treatment of a proliferative, autoimmune or infections disease, for example cancer; and a conjugate according to the claims for use in a method of treating a patient which comprises administering a pharmaceutically-effective amount of such a conjugate or pharmaceutical composition to a patient. Particular conditions for which the present invention finds utility include for example leukaemia, including non-Hodgkin's Lymphoma, acute myelogenous leukaemia, multiple myeloma, lymphocytic leukaemias, and chronic myelogenous leukaemia; gastric cancer; breast cancer; ovarian cancer; liver cancer; intestinal cancer; colon cancer; renal cancer, for example renal cell carcinoma; lung cancer, for example small cell lung cancer; melanoma; bladder cancer; and sarcomas.

Conjugates of the present invention demonstrate a number of important advantages, the existence of which could not have been predicted. Compared with equivalent drug-antibody conjugates prepared using maleimide reagents, as currently used in commercially available conjugates, they demonstrate significantly increased stability. Further, their method of synthesis leads to a product with a significantly improved homogeneity in respect of drug-antibody ratio, compared with the use of maleimide. Homogeneity is advantageous for drug substances for regularity of production of the drug substance. A process which generates a greater yield of a single DAR species will be cheaper through greater efficiency. A drug product of a single DAR species produced by purification of that DAR species from a heterogeneous mixture would be prohibitively expensive to produce in large quantities.

Further, a single DAR species will demonstrate more predictable pharmacokinetics, safety, toxicity and tolerability as all the drug substance should be metabolised in a similar manner, giving the same products, as opposed to a mixed DAR substance which may be metabolised differently or at different rates, giving more heterogeneous break down products.

The following Examples illustrate the invention. Subject-matter not encompassed under the scope of the claims does not form part of the invention.

Example 1: Synthesis of valine-alanine-paraaminobenzyl-aminohexoanoic-maytansine (val-ala-PAB-AHX-DM1) reagent 1 possessing a 24 repeat unit PEG with terminal bis-sulfone functionality.

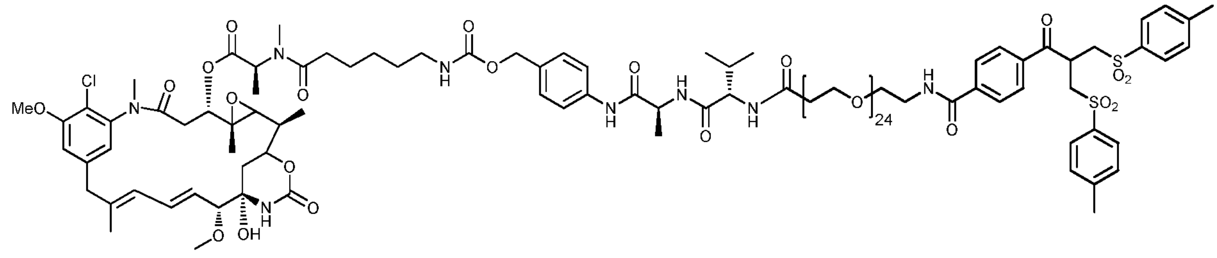

**1**

Step 1: Conjugation of 1-oxo-1-(4-(3-tosyl-2-(tosylmethyl)propanoyl)phenyl)-PEG acid (bis-sulfone-PEG(24 unit)-$CO_2H$)

O-(6-Chlorobenzotriazol-1-yl)-N,N,N',N'-tetramethyluronium hexafluorophosphate (HCTU) (29 mg) was added to a stirred solution of bis-sulfone-PEG(24)-$CO_2H$ (75 mg, prepared from $NH_2$-PEG(24)-$CO_2H$ using a method derived from Nat. Prot., Vol 1, No. 4, 2006) in anhydrous dimethylformamide (5.8 mL) and stirred for 20 min. Val-ala-PAB-AHX-DM1 (100 mg, Concortis Biosystems Corp.) was added and the reaction mixture was stirred at 30 °C. After 24 h, an additional amount of HCTU (2 mg) was added and the reaction mixture was stirred at 30 °C. After a further 16 h, volatiles were removed *in vacuo* and the solid residue was purified by column chromatography eluting with dichloromethane-methanol (97:3 v/v +5 drops of AcOH/50 mL solvent, then 96:4 v/v +5 drops of AcOH/50 mL solvent), the solvent was removed under vacuum and the bis-sulfone-PEG(24)-val-ala-PAB-AHX-DM1 **1** was isolated as a pale yellow oil (55 mg). m/z M+Na+ 2716; diagnostic signals for $^1H$ NMR $\partial_H$ (600 MHz $CDCl_3$) 1.28 (6H, dd, valine $CH(CH_3)_2$), 1.45 (3H, d, alanine $CH\text{-}CH_3$) 2.49 (6H, s, $CH_3$-Ar), 3.45-3.75 (m, PEG and $CH_2$-Ts), 5.62 (1H, dd, C=CH-CH-C=C*H*), 6.41 (1H, dd, C=CH-C*H*-C=CH), 6.65 (2H, s, $CH_2$-Ar), 7.37 (4H, d, Ts), 7.63 (2H, d, Ar), 7.66 (4H, d, Ts), 7.85 (2H, d, Ts).

Example 2. Synthesis of valine-alanine-paraaminobenzyl-aminohexoanoic-maytansine (val-ala-PAB-AHX-DM1) reagent **2** possessing a 5 kDa repeat unit PEG with terminal bis-sulfone functionality.

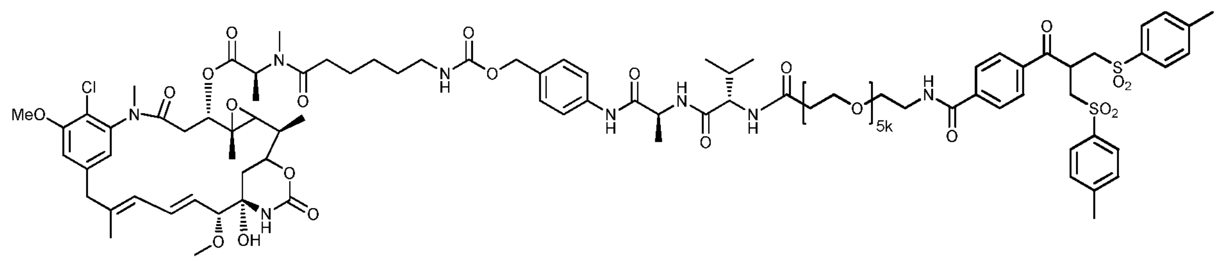

**2**

Step 1: Conjugation of 3-(2-(4-(3-tosyl-2-(tosylmethyl)propanoyl)phenyl benzamido)ethoxy)PEG acid (bis-sulfone-PEG(5 kDa)-$CO_2H$)

1-[Bis(dimethylamino)methylene]-1H-1,2,3-triazolo[4,5-b]pyridinium 3-oxid hexafluorophosphate (HATU) (10 mg) was added to a stirred solution of the known bis-sulfone-PEG(5 kDa)-$CO_2H$ (56 mg, prepared from $NH_2$-PEG(5 kDa)-$CO_2H$ using a method derived from Nat. Prot., Vol 1, No. 4, 2006) in anhydrous dimethylformamide (0.5 mL) when a solution of val-ala-PAB-AHX-DM1 (15.8 mg, Concortis Biosystems Corp.) in dimethylformamide (0.5 mL) was added followed by N-methyl morpholine (4.4 $\mu$L) and the reaction mixture was stirred at room temperature. After 24 h, a further amount of HATU (10 mg) and N-methyl morpholine (4.4 $\mu$L) was added and the reaction was stirred at room temperature. After a further 19 h, volatiles were removed *in vacuo* and the solid residue was purified by reverse-phase C18-column chromatography eluting with buffer A (v/v): water:5% acetonitrile:0.1% TFA and buffer B (v/v): 0.1% TFA in acetonitrile (100:0 v/v to 0:100), the organic solvent was removed under vacuum and the aqueous solvent was removed by lyophilisation and the bis-sulfone-PEG(5 kDa)-val-ala-PAB-AHX-DM1 **2** was isolated as a colourless film (10.6 mg). m/z M+Na+ 6380.

Example 3. Conjugation of bis-sulfone-PEG(24)-val-ala-PAB-AHX-DM1 1 to an antibody (trastuzumab)

To a solution of antibody (2.25 mg) (Herceptin®) at 5 mg/mL in 20 mM sodium phosphate, pH 7.5, 150 mM NaCl and 20 mM EDTA was added a 5 mM TCEP solution (18 μL) and the resulting mixture was incubated at 40 °C for 1 h. The reduced antibody solution (0.468 mL, at 4.8 mg/mL) was diluted to 3.33 mg/mL with 20 mM sodium phosphate buffer, pH 7.5, 150 mM NaCl and 20 mM EDTA. To the reduced antibody, a solution bis-sulfone-PEG(24)-val-ala-PAB-AHX-DM1 reagent **1** in DMF (75 μL at 3.23 mg/mL) was added and the resulting solution was mixed and incubated at 22 °C for 22 h. The reaction was quenched by addition of 50 mM *N*-acetyl-L-cysteine (36 μL) and incubated at 22 °C for a further 1 h. The final reaction mixture (786 μL) was buffer exchanged into PBS ×1 using a 5 mL Zeba™ spin column (Pierce). The reaction mixture was analysed by Hydrophobic Interaction Chromatography (HIC) using a TOSOH TSK-gel Butyl-NPR column. The method consisted of a linear gradient from 100% buffer A (50 mM sodium phosphate pH 7.0, 1.5 M ammonium sulfate) to 100 % buffer B (50 mM sodium phosphate pH 7.0, 20% isopropanol) in 30 mins. Detection was carried out by following UV absoption at 248 and 280 nm. Species were separated according to amount of drug loaded and characterised according to the ratio of UV absorbance maxima at 248 and 280 nm. The area for each DAR variant was determined and plotted as a bar chart and the result is shown in Figure 1. The major product was the DAR=4 conjugate (65%).

Example 4. Conjugation of bis-sulfone-PEG(24)-val-ala-PAB-AHX-DM1 reagent **1** to a Fab

To a solution of a Fab (5 mg, derived from the papain digestion of trastuzumab) at 2.59 mg/mL in PBS was added 19.3 μL of 1 M DTT. The reduction mixture was incubated at 22 °C for 1 h. After incubation, the mixture was buffer exchanged into 20 mM sodium phosphate, pH 7.4, 150 mM NaCl and 20 mM EDTA using a 5 mL Zeba™ Spin Desalting column. The reduced Fab solution (1.9 mL, at 2.58 mg/mL) was diluted to 2.22 mg/mL with 20 mM sodium phosphate buffer, pH 7.4, 150 mM NaCl and 20 mM EDTA. To the reduced Fab (2.12 mL at 2.22 mg/mL), a solution of bis-sulfone-PEG(24)-val-ala-PAB-AHX-DM1 reagent **1** in 50% DMSO and 50% 20 mM sodium phosphate buffer, pH 7.4, 150 mM NaCl and 20 mM EDTA (235 μL at 1.62 mg/mL) was added and the resulting solution was mixed and incubated at 22 °C for 18 h. The reaction mixture was then analysed by SDS-PAGE. The conjugation mixture was then purified by preparative HIC using a 5 mL HiTrap™ Phenyl HP HIC column. The purified Fab-reagent **1** conjugate was analysed by RP-HPLC (Figure 2) and SDS-PAGE (Figure 3). The RP-HPLC analysis was conducted on a VariTide RPC 250 x 4.6 mm column (Agilent Technologies) and as shown in Figure 2 the Fab-AHX-DM1 conjugate product ran as a single main peak (92% by area) showing the conjugate to be highly homogeneous. For SDS-PAGE analysis, samples were run on a 4-12% Bis-Tris gel in MES running buffer at 200 V for 35 min. Novex® Sharp Pre-stained Standard was used as the protein markers. NuPAGE® LDS Sample Buffer (4X) was used as the sample buffer and the gel was stained with InstantBlue™ protein stain. Densitometry was analysed by ImageQuant LAS 4000 to give a purity reading for each lane which is displayed as a percentage on the gel in Figure 4. One μg of sample (based on Fab) was loaded in each lane, i.e., for each sample. In Figure 4, the lane labelled M is the protein standards, the lane labelled 1 is the starting Fab the lane labelled 2 is the starting Fab after treatment with DTT, the lane labelled 3 is the purified Fab-AHX-DM1 conjugate and the lane labelled 4 is the purified Fab-AHX-DM1 conjugate after treatment with DTT. Both the Fab and conjugate were treated with DTT in the same way, i.e, 10 mM final DTT concentration for 1 h at room temperature.

Example 5. Analysis of antibody drug conjugates (ADCs) by *in vitro* cell viability assay

The *in vitro* efficacy of the antibody and Fab conjugates prepared in Examples 3 and 4 respectively were determined by measuring the inhibitory effect on cell growth of HER-2 receptor over-expressing cancer cell lines.

Loss of tumour cell viability following treatment with cytotoxic drugs or ADCs *in vitro* can be measured by growing cell lines in the presence of increasing concentrations of drugs or ADCs and quantifying the loss of proliferation or metabolic activity using Cell-Titer Glo® Luminescence reagent (Promega Corp. Technical Bullettin TB288; Lewis Phillips G.D, Cancer Res 2008; 68:9280-9290). The protocol describes cell seeding, drug treatment and determination of the cell viability in reference to untreated cells based on ATP synthesis, which is directly related to the number of cells present in the well.

HER2-positive SK-BR-3 and HER2-negative MCF-7 cells were trypsinised with 3 mL Trypsin EDTA for 5-15 min. Trypsinisation was stopped by adding 10 mL complete medium, and cells were transferred to a 50 mL Falcon tube. Cells were counted using a Neubauer haemocytometer and adjusted to a cell density of $3x10^4$/mL for MCF-7 and $5x10^4$/mL for SK-BR-3 respectively. Cells were seeded (100 μL/well) into poly-D-lysine coated opaque-walled 96-well plates and incubated for 24 h at 37°C and 5% $CO_2$. Tumour cell lines SK-BR-3 (ATCC-HTB-30) and MCF-7 (ATCC-HTB-22) were purchased from the American Type Culture Collection. SK-BR-3 cells were grown in McCoy's 5A medium (Life Technologies®), 10% fetal bovine serum, 100 u/mL Penicillin and 100 μg/mL Streptomycin. MCF-7 cells were grown in Minimal Essential Medium (Life Technologies®), 10% fetal bovine serum, 100 u/mL Penicillin and 100 μg/mL

Streptomycin. Methods for cell culture were derived from product information sheets for ATCC and references quoted therein, for example, Culture of Animal Cells: A Manual of Basic Technique by R. Ian Freshney 3rd edition, published by Alan R. Liss, N.Y. 1994, or 5th edition published by Wiley-Liss, N.Y. 2005.

Serial dilutions of ADC or free drug (AHX-DM1), were made in triplicate by pipetting across a 96 well plate from columns 3-10 using the relevant cell culture medium as a diluent. The HER2-positive cell line SK-BR-3, was treated with drug concentrations of 40-0.018 nM using 2-fold dilutions. The HER2-negative cell line MCF-7 was treated with drug concentrations of 200-0.1 nM (free AHX-DM1) or 400-0.2 nM (AHX-DM1 conjugates), respectively using 3 fold dilutions. Cells were then incubated with the drug (total volume 200 μL/well), at 37 °C and 5% $CO_2$ for a further 96 h.

The cell viability assay was carried out using the Cell-Titer Glo® Luminescence reagent, as described by the manufacturer's instructions, (Promega Corp. Technical Bulletin TB288; Lewis Phillips G.D, Cancer Res 2008; 68:9280-9290). Incubation times, e.g. cell lysis and incubation with luminescent reagent, were extended to 3 min and 20 min respectively, for optimal luminescent signal.

Luminescence was recorded using a plate reader (e.g. MD SpectramaxM3 plate reader), and data subsequently analysed using a four parameter non-linear regression model.

Figure 4. Cell viability responses to treatment with either Fab- reagent **1** conjugate or Antibody-reagent **1** conjugate within SKBR-3 or MCF-7 cells.

Viability is expressed as % of untreated cells. The % viability (Y-axis) is plotted against the logarithm of drug concentration in nM (x-axis) to determine the IC50 values for all conjugates as well as free drug.

As shown in Figure 4 and Table 1, both the Antibody-val-ala-PAB-AHX-DM1 and Fab-val-ala-PAB-AHX-DM1 conjugates are efficiently inhibiting the proliferation of HER2-positive SK-BR-3 cells. In HER2-negative MCF-7 cells, no growth inhibition is observed following Fab-val-ala-PAB-AHX-DM1 or antibody-val-ala-PAB-AHX-DM1 treatment at the concentrations tested, confirming the specificity of the conjugates.

**Table 1**

| Sample | SK-BR-3 $IC_{50}$ (nM) | MCF-7 $IC_{50}$ (nM) |
|---|---|---|
| Fab- reagent **1** conjugate (DAR=1) | 0.33 | N/A |
| Antibody-reagent **1** conjugate (DAR=1) | 0.12 | N/A |
| AHX-DM1 | 3.4 | 36.26 |

Example 6. Synthesis of reagent **3**

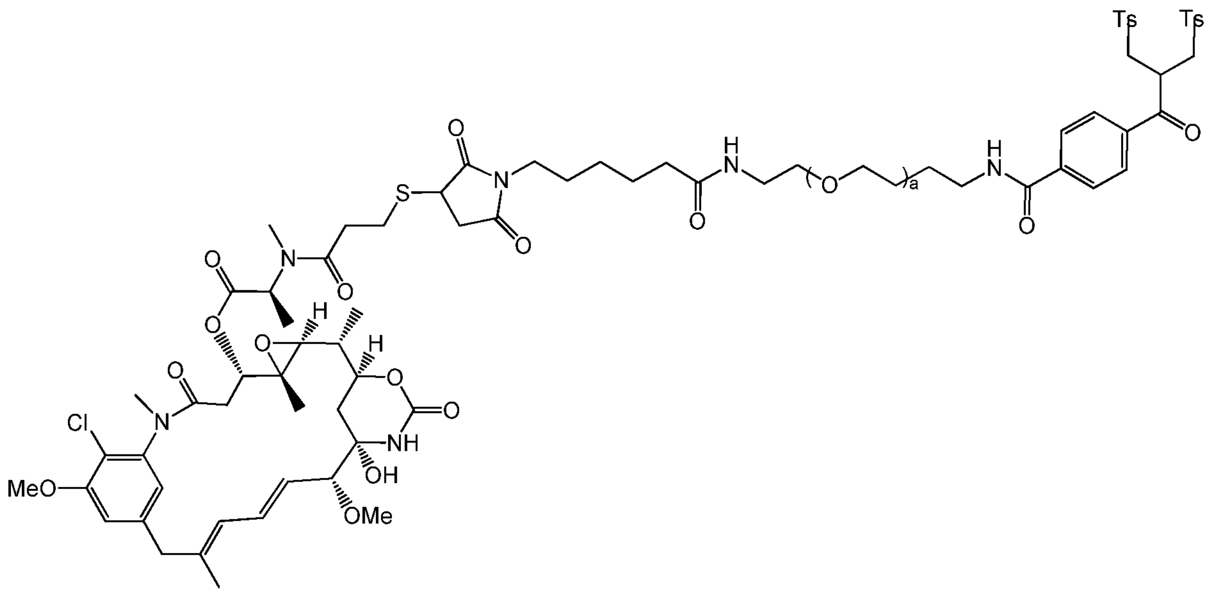

**3**

Step 1: Conjugation of 4-[2,2-bis[(p-tolylsulfonyl)-methyl]acetyl]benzoic acid-N-hydroxy succinimidyl ester (bis-sulfone) to *O*-(2-aminoethyl)-*O*'-[2-(Boc-amino)ethyl]decaethylene glycol

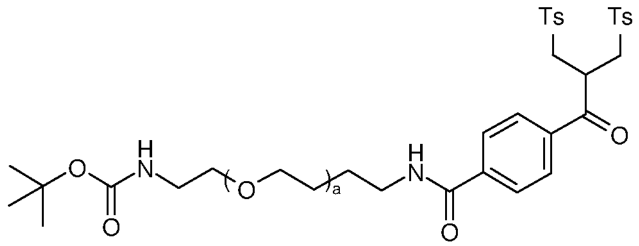

**4**

*O*-(2-aminoethyl)-*O*'-[2-(Boc-amino)ethyl]decaethylene glycol (Sigma Aldrich) is dissolved in dichloromethane. Under stirring, 4-[2,2-bis[(p-tolylsulfonyl)-methyl]acetyl]benzoic acid-N-hydroxy succinimidyl ester (Nature Protocols, 2006, 1(54), 2241-2252) is added and the mixture is stirred under an argon atmosphere until the reaction is complete. Volatiles are removed *in vacuo* and the residue is purified to give **4**.

Step 2: Deprotection of the Boc-protecting group.

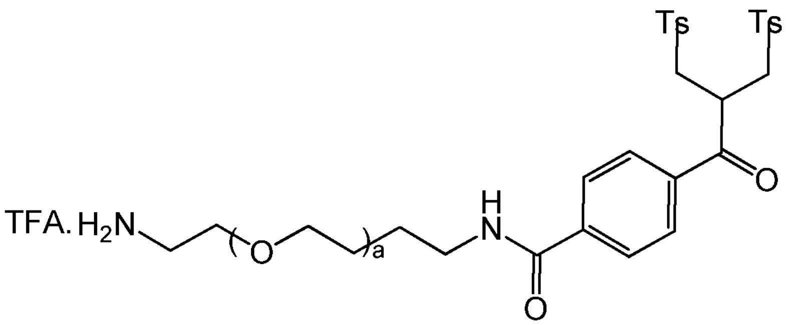

**5**

To a stirred solution of the product of step 1 in dichloromethane is added trifluoroacetic acid and the resulting solution is stirred until Boc deprotection is complete. Volatiles are removed *in vacuo* and the residue is purified to give **5**.

Step 3: Conjugation of DM1 to 6-Maleimidohexanoic acid

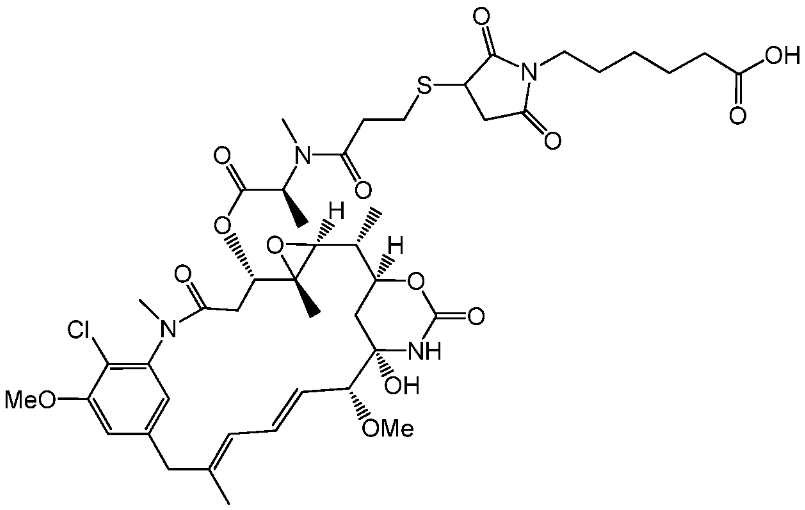

**6**

A mixture of DM1 (Widdison et al, J Med. Chem., 2006, 49(14), p4392-4408), WO2009/134976), 6-Maleimidohexanoic acid (Sigma Aldrich) and potassium phosphate buffer in THF/water is stirred until the reaction proceeds to completion. The volatile components are removed *in vacuo* and the residue purified to give **4**.

Step 4: Reaction of **5** with **6**.

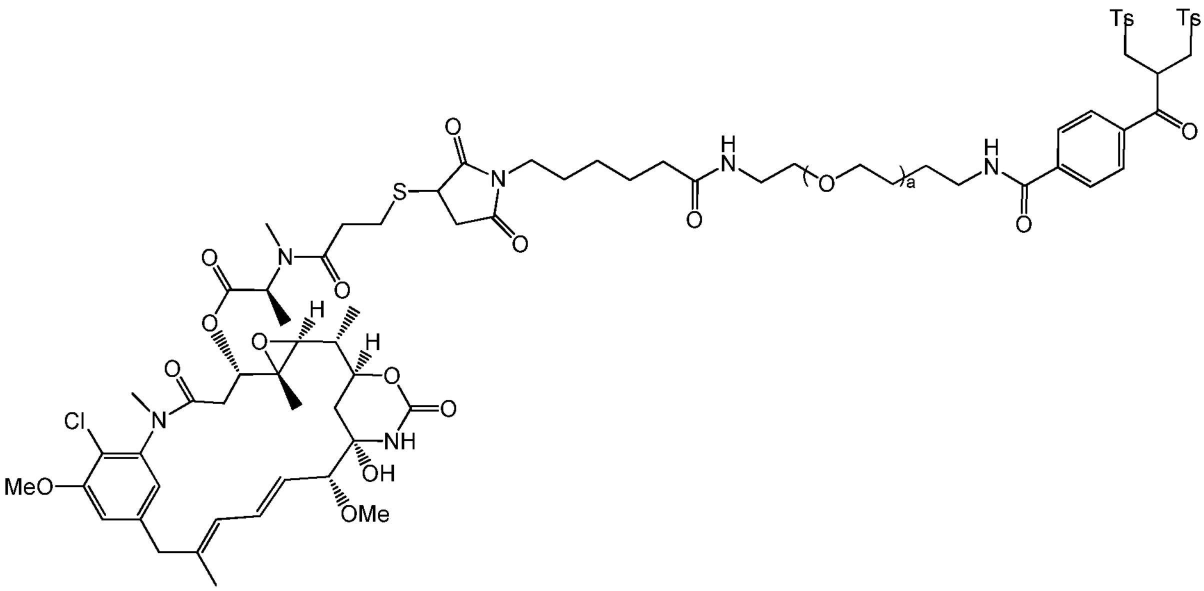

**3**

A mixture of **4**, DMAP and EDCI are stirred at room temperature in DMF for 30 minutes. **5** is added and the mixture is stirred until the reaction proceeds to completion. The volatile components are removed *in vacuo* and the residue purified to give **3**.

## Claims

**1.** A maytansine-containing conjugate of the general formula:

$$(((D_q\text{-}Lk^1)_m\text{-}P)_p\text{-}Lk^2\text{-}Lk^3)_n\text{-}Ab \qquad (I)$$

in which D represents a maytansine moiety;
q represents an integer from 1 to 10;
$Lk^1$ represents a linker;
m represents an integer from 1 to 10;
P represents a bond or a z-valent group $-P^1\text{-NH-}$ where z is from 2 to 11 and $P^1$ is a group containing at least one ethylene unit $-CH_2\text{-}CH_2-$ or ethylene glycol unit $-O\text{-}CH_2\text{-}CH_2-$;
p represents an integer from 1 to 10;
$Lk^2$ represents a bond or a y-valent linker where y is from 2 to 11 and which consists of from 1 to 9 aspartate and/or glutamate residues;
$Lk^3$ represents a linker of the general formula:

$$\text{-CO-Ph-X-Y-} \qquad (II)$$

in which Ph is an optionally substituted phenyl group; X represents a CO group or a CH.OH group; and Y represents a group of formula:

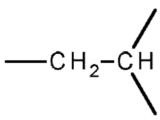
(III) or
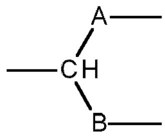
(IV)

in which each of A and B represents a $C_{1-4}$alkylene or alkenylene group;
Ab represents a binding protein or peptide capable of binding to a binding partner on a target, said binding protein or peptide being bonded to $Lk^3$ via two sulfur atoms derived from a disulfide bond in the binding protein or peptide; and
n represents an integer from 1 to s where s is the number of disulfide bonds present in the binding protein or peptide prior to conjugation to $Lk^3$;
the meanings of m, n, p, q, y and z being chosen such that the conjugate contains from 1 to 10 D groups.

**2.** A conjugate as claimed in claim 1, wherein the maytansine moiety comprises

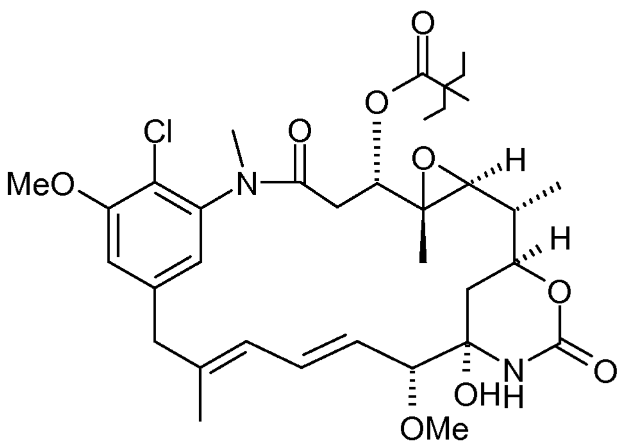
(C).

3. A conjugate as claimed in any one of the preceding claims, in which $Lk^1$ is a degradable linker.

4. A conjugate as claimed in claim 3, in which $Lk^1$ includes one of the following groups:

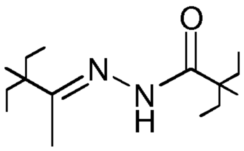 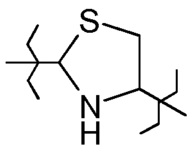 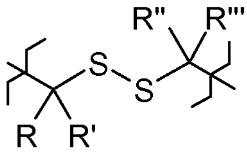 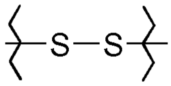

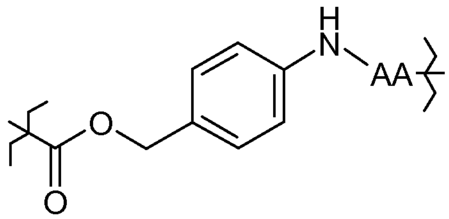 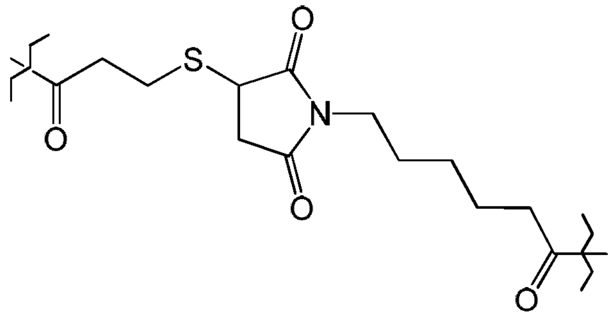

in which each of R, R', R" and R''' represents a hydrogen atom or an alkyl group and AA represents a protease-specific amino acid sequence.

5. A conjugate as claimed in claim 4, in which $Lk^1$ includes:

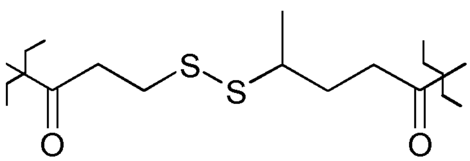 (Vb) 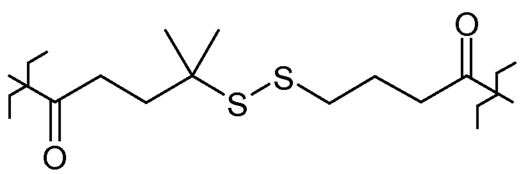 (Vc),

or

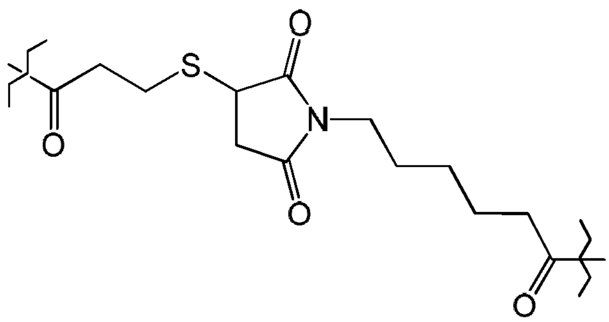 .

6. A conjugate as claimed in claim 1 or claim 2, in which q is an integer from 2 to 10 and $Lk^1$ is a multivalent linker incorporating one or more aspartate or glutamate residues.

7. A conjugate as claimed in any one of claims 1 to 6, in which P represents a bond, or P represents $-P^1-NH-$ wherein $P^1$ contains from 2 to 10 ethylene glycol units.

8. A conjugate as claimed in any one of claims 1 to 6, in which $P^1$ represents polyethylene glycol.

9. A conjugate as claimed in any one of claims 1 to 8, in which the phenyl group Ph in $Lk^3$ is unsubstituted.

**10.** A conjugate as claimed in any one of claims 1 to 9, in which Y has the formula:

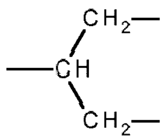

**11.** A conjugate as claimed in any one of claims 1 to 10, in which Ab represents a full length antibody or an antibody fragment comprising an antigen-binding region of the full length antibody.

**12.** A conjugate as claimed in claim 11, in which Ab represents IgG1 or IgG4 or a fragment of IgG1 or IgG4.

**13.** A process for the preparation of a conjugate as claimed in any one of claims 1 to 12, which comprises reducing one or more disulfide bonds in a binding protein and subsequently reacting with a conjugating reagent of the general formula:

$$((D_q\text{-}Lk^1)_m\text{-}P)_p\text{-}Lk^2\text{-}Lk^{3a} \qquad \text{(VIII)}$$

in which D, $Lk^1$, P, $Lk^2$ and m, p and q have the meanings given in claim 1, and $Lk^{3a}$ represents a group of formula:

$$\text{-CO-Ph-}X^a\text{-}Y^a \qquad \text{(IX)}$$

in which Ph has the meaning given in claim 1, $X^a$ represents a CO group, and $Y^a$ represents a group:

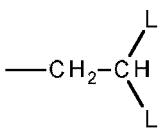
(X),
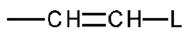
(XI),
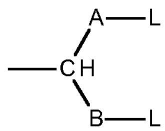
(XII)

or

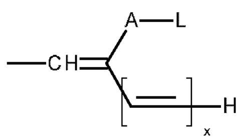
(XIII)

in which A and B have the meanings given in claim 1, each L independently represents a leaving group, and x represents an integer from 1 to 4, to produce a conjugate of formula I in which X represents CO; and optionally reducing said initially-formed CO group X to give a conjugate having a CH.OH group X.

**14.** A process as claimed in claim 13, in which $Y^a$ represents:

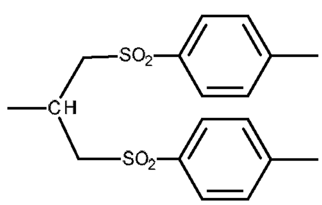
(XIIa)

**15.** A compound of the general formula:

$$((D_q\text{-}Lk^1)_m\text{-}P)_p\text{-}Lk^2\text{-}Lk^{3a} \qquad (VIII)$$

in which D has the meaning given in any one of claims 1 to 3; $Lk^1$ has the meaning given in any one of claims 1 and 4 to 7; P has the meaning given in any one of claims 1, 8 and 9; $Lk^2$, m, p and q have the meanings given in claim 1, and $Lk^{3a}$ represents a group of formula:

$$\text{-CO-Ph-}X^a\text{-}Y^a \qquad (IX)$$

in which Ph has the meaning given in either claim 1 or claim 10, $X^a$ represents a CO group, and $Y^a$ represents a group:

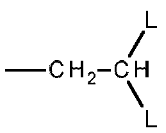 (X), 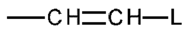 (XI), 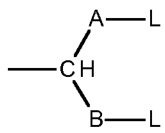 (XII)

or

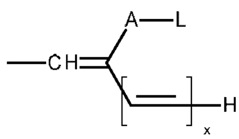 (XIII)

in which A and B have the meanings given in claim 1, each L independently represents a leaving group, and x represents an integer from 1 to 4.

**16.** A compound as claimed in claim 15, in which $Y^a$ represents:

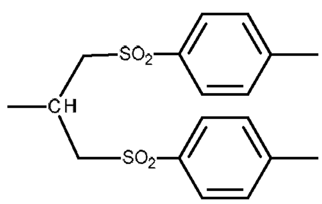 (XIIa)

**17.** A pharmaceutical composition comprising a conjugate as claimed in any one of claims 1 to 12, together with a pharmaceutically acceptable carrier, optionally together with an additional therapeutic agent.

**18.** A conjugate as claimed in any one of claims 1 to 12 or a composition as claimed in claim 17 for use in therapy.

## Patentansprüche

**1.** Maytansin-enthaltendes Konjugat der allgemeinen Formel:

$$(((D_q\text{-}Lk^1)_m\text{-}P)_p\text{-}Lk^2\text{-}Lk^3)_n\text{-}Ab \qquad (I)$$

in der D einen Maytansin-Einheit darstellt;

q eine ganze Zahl von 1 bis 10 darstellt;
$Lk^1$ einen Linker darstellt;
m eine ganze Zahl von 1 bis 10 darstellt;
P eine Bindung oder eine z-wertige Gruppe $-P^1-NH-$ darstellt, wobei z für 2 bis 11 steht und $P^1$ eine Gruppe ist, die mindestens eine Ethylen-Einheit $-CH_2-CH_2-$ oder eine Ethylenglykol-Einheit $-O-CH_2-CH_2-$ enthält;
p eine ganze Zahl von 1 bis 10 darstellt;
$Lk^2$ eine Bindung oder einen y-wertigen Linker darstellt, wobei y für 2 bis 11 steht und der Linker aus 1 bis 9 Aspartat- und / oder Glutamatresten besteht;
$Lk^3$ einen Linker der allgemeinen Formel:

$$-CO-Ph-X-Y- \quad (II)$$

darstellt, in dem Ph eine gegebenenfalls substituierte Phenylgruppe ist; X eine CO-Gruppe oder eine CH.OH-Gruppe darstellt; und Y eine Gruppe der Formel:

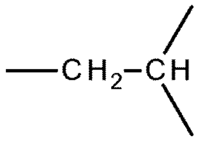
(III)
oder
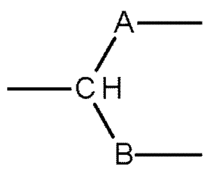
(IV)

darstellt,

in der A und B jeweils eine $C_{1-4}$- Alkylen- oder Alkenylengruppe darstellt;
Ab ein Bindungsprotein oder -peptid darstellt, das fähig ist, an einen Bindungspartner an einem Ziel zu binden, wobei das Bindungsprotein oder -peptid über zwei Schwefelatome, die von einer Disulfidbindung in dem Bindungsprotein oder -peptid stammen, an $Lk^3$ gebunden ist; und
n eine ganze Zahl von 1 bis s darstellt, wobei s für die Anzahl der Disulfidbindungen steht, die vor der Konjugation mit $Lk^3$ in dem Bindungsprotein oder -peptid vorhandenen ist;

wobei die Bedeutungen von m, n, p, q, y und z so gewählt sind, dass das Konjugat 1 bis 10 Gruppen D enthält.

**2.** Das Konjugat, wie in Anspruch 1 beansprucht, wobei die Maytansin-Einheit umfasst:

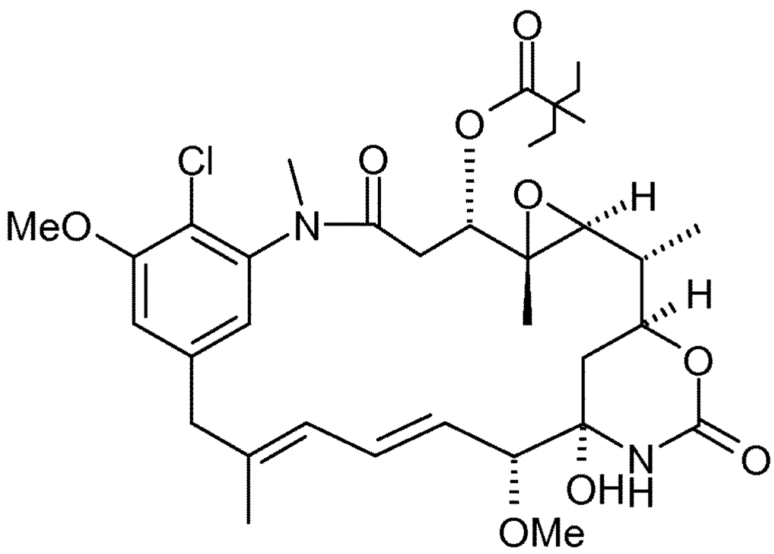
(C).

**3.** Das Konjugat, wie in einem der vorhergehenden Ansprüche beansprucht, in dem $Lk^1$ ein abbaubarer Linker ist.

4. Das Konjugat, wie in Anspruch 3 beansprucht, in dem $Lk^1$ eine der folgenden Gruppen:

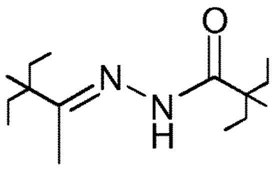
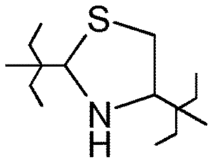
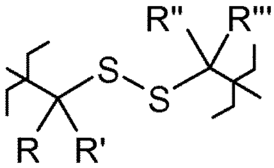
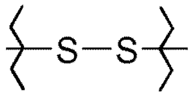

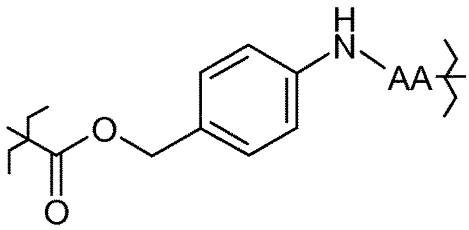
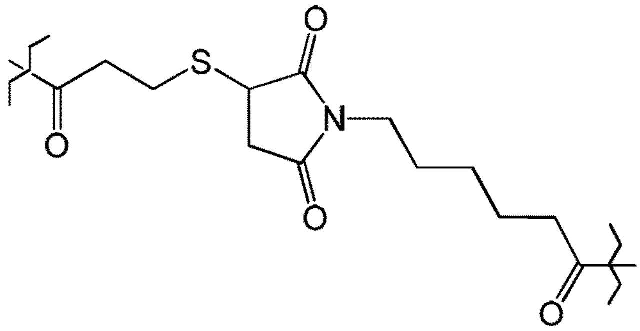
,

in denen R, R’, R'' und R’’’ jeweils ein Wasserstoffatom oder eine Alkylgruppe darstellt und AA eine Protease-spezifische Aminosäuresequenz darstellt, beinhaltet.

5. Das Konjugat, wie in Anspruch 4 beansprucht, in dem $Lk^1$ beinhaltet:

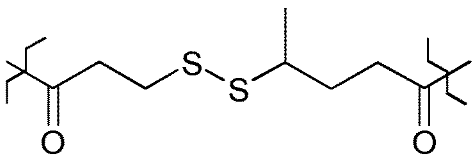
(Vb)
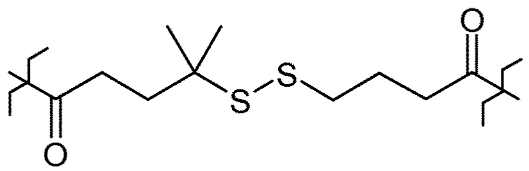
(Vc),

oder

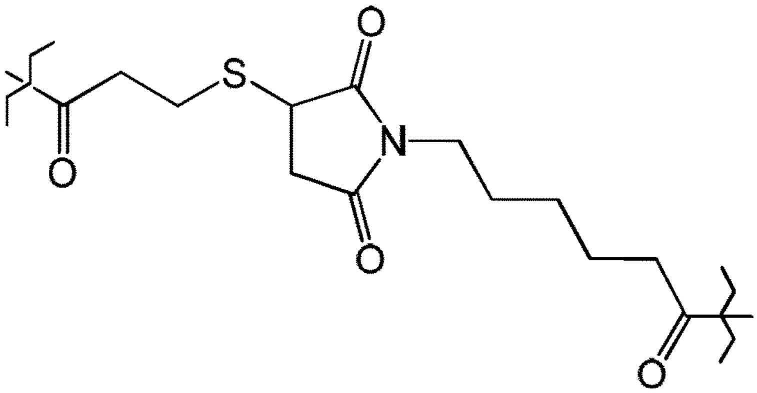
.

6. Das Konjugat, wie in Anspruch 1 oder Anspruch 2 beansprucht, in dem q für eine ganze Zahl von 2 bis 10 steht und $Lk^1$ ein mehrwertige Linker ist, in den ein oder mehrere Aspartat- oder Glutamatrest(e) eingebaut ist / sind.

7. Das Konjugat, wie in einem der Ansprüche 1 bis 6 beansprucht, in dem P eine Bindung darstellt, oder P für $-P^1-NH-$ steht, wobei $P^1$ von 2 bis 10 EthylenglykolEinheiten enthält.

8. Das Konjugat, wie in einem der Ansprüche 1 bis 6 beansprucht, in dem $P^1$ ein Polyethylenglykol darstellt.

**9.** Das Konjugat, wie in einem der Ansprüche 1 bis 8 beansprucht, in dem die Phenylgruppe Ph in $Lk^3$ unsubstituiert ist.

**10.** Das Konjugat, wie in einem der Ansprüche 1 bis 9 beansprucht, in dem Y die Formel:

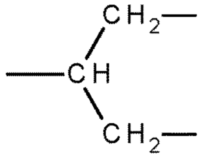

aufweist.

**11.** Das Konjugat, wie in einem der Ansprüche 1 bis 10 beansprucht, in dem Ab einen Antikörper voller Länge darstellt oder ein Antikörperfragment, das eine Antigen-bindende Region des Antikörpers voller Länge umfasst.

**12.** Das Konjugat, wie in Anspruch 11 beansprucht, in dem Ab ein IgG1 oder ein IgG4 darstellt oder ein Fragment von einem IgG1 oder einem IgG4.

**13.** Verfahren zur Herstellung eines Konjugats, wie in einem der Ansprüche 1 bis 12 beansprucht, welches umfasst: das Reduzieren von einer oder mehreren Disulfidbindung(en) in einem Bindungsprotein und das anschließende Umsetzen mit einem Konjugationsreagenz der allgemeinen Formel:

$$((D_q\text{-}Lk^1)_m\text{-}P)_p\text{-}Lk^2\text{-}Lk^{3a} \qquad \text{(VIII)}$$

in dem D, $Lk^1$, P, $Lk^2$ sowie m, p und q die Bedeutungen haben, die in Anspruch 1 angegeben ist, und $Lk^{3a}$ eine Gruppe der Formel:

$$\text{-CO-Ph-}X^a\text{-}Y^a \qquad \text{(IX)}$$

darstellt, in der Ph die in Anspruch 1 angegebene Bedeutung hat, $X^a$ eine Gruppe CO darstellt; und $Y^a$ eine Gruppe:

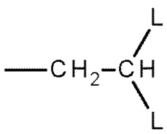 (X), 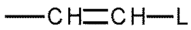 (XI), 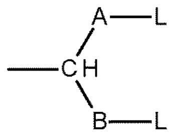 (XII)

oder

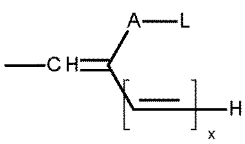 (XIII)

darstellt, in der A und B die Bedeutungen haben, die in Anspruch 1 angegeben ist, jedes L jeweils unabhängig eine Abgangsgruppe darstellt, und x eine ganze Zahl von 1 bis 4 darstellt, um ein Konjugat der Formel (I) herzustellen, in dem X für CO steht; und gegebenenfalls das Reduzieren der anfänglich gebildeten CO-Gruppe an der Position X, um ein Konjugat zu erhalten, das an der Position X eine Gruppe CH.OH aufweist.

**14.** Das Verfahren, wie in Anspruch 13 beansprucht, in dem $Y^a$ darstellt:

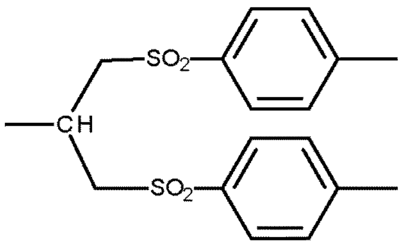

(XIIa).

**15.** Verbindung der allgemeinen Formel:

$$((D_q\text{-}Lk^1)_m\text{-}P)_p\text{-}Lk^2\text{-}Lk^{3a} \qquad \text{(VIII)}$$

in der D die Bedeutung hat, die in einem der Ansprüche 1 bis 3 angegeben ist; $Lk^1$ die Bedeutung hat, die in einem der Ansprüche 1 und 4 bis 7 angegeben ist; P die Bedeutung hat, die in einem der Ansprüche 1, 7 [8] und 8 [9] angegeben ist; $Lk^2$, m, p und q die Bedeutungen haben, die in Anspruch 1 angegeben ist; und $Lk^3$ eine Gruppe der Formel:

$$\text{-CO-Ph-}X^a\text{-}Y^a \qquad \text{(IX)}$$

darstellt, in der Ph entweder die Bedeutung hat, die entweder in Anspruch 1 oder in Anspruch 10 angegeben ist; $X^a$ eine CO-Gruppe darstellt; und $Y^a$ eine Gruppe:

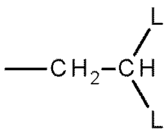

(X),

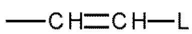

(XI),

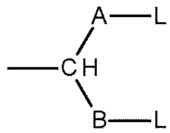

(XII)

oder

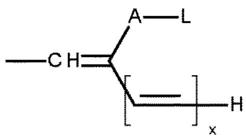

(XIII)

darstellt,

in der A und B die Bedeutungen haben, die in Anspruch 1 angegeben ist, jedes L jeweils unabhängig eine Abgangsgruppe darstellt, und x eine ganze Zahl von 1 bis 4 darstellt.

**16.** Die Verbindung, wie in Anspruch 15 beansprucht, in der $Y^a$ darstellt:

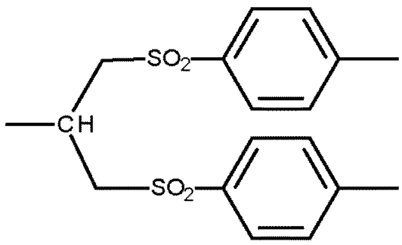

(XIIa) .

17. Pharmazeutische Zusammensetzung, umfassend ein Konjugat, wie in einem der Ansprüche 1 bis 12 beansprucht, zusammen mit einem pharmazeutisch akzeptablen Träger, gegebenenfalls zusammen mit einem zusätzlichen therapeutischen Mittel.

18. Das Konjugat, wie in einem der Ansprüche 1 bis 12 beansprucht, oder eine Zusammensetzung, wie in Anspruch 17 beansprucht, zur Verwendung bei einer Therapie.

## Revendications

1. Conjugué contenant de la maytansine de formule générale :

$$(((D_q\text{-}Lk^1)_m\text{-}P)_p\text{-}Lk^2\text{-}Lk^3)_n\text{-}Ab \qquad (I)$$

dans laquelle D représente un groupement maytansine ;
q représente un nombre entier de 1 à 10 ;
$Lk^1$ représente un lieur ;
m représente un nombre entier de 1 à 10 ;
P représente une liaison ou un groupe z-valent $-P^1$-NH-où z vaut 2 à 11 et $P^1$ est un groupe contenant au moins une unité éthylène $-CH_2-CH_2-$ ou une unité éthylèneglycol $-O-CH_2-CH_2-$ ;
$Lk^2$ représente une liaison ou un lieur y-valent où y vaut 2 à 11 et qui est constitué de 1 à 9 résidus aspartate et/ou glutamate ;
$Lk^3$ représente un lieur de formule générale

$$\text{-CO-Ph-X-Y-} \qquad (II)$$

dans laquelle Ph est un groupe phényle éventuellement substitué ; X représente un groupe CO ou un groupe CH.OH ; et Y représente un groupe de formule :

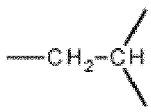 (III) ou 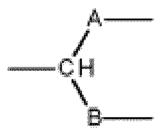 (IV)

dans laquelle chacun de A et B représente un groupe alkylène en $C_1$ à $C_4$ ou alcénylène,
Ab représente une protéine ou un peptide de liaison capable de se lier à un partenaire de liaison sur une cible, ladite protéine ou peptide de liaison étant lié à $Lk^3$ via deux atomes de soufre dérivés d'une liaison disulfure dans la protéine ou le peptide de liaison ; et
n représente un nombre entier de 1 à s où s est le nombre de liaisons disulfure présentes dans la protéine ou le peptide de liaison avant la conjugaison à $Lk^3$ ;
les significations de m, n, p, q, y et z étant choisies de telle manière que le conjugué contient 1 à 10 groupes D.

2. Conjugué selon la revendication 1, dans lequel le groupement maytansine comprend

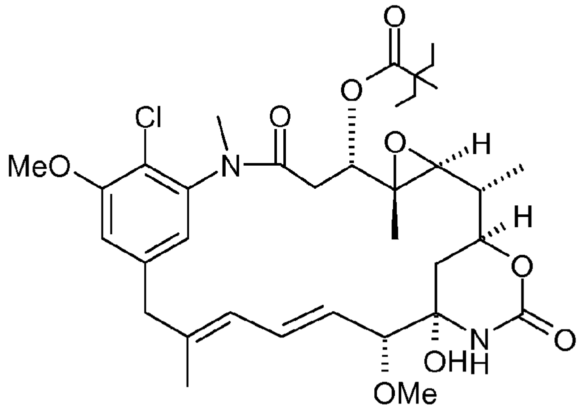

(C).

3. Conjugué selon l'une quelconque des revendications précédentes, dans lequel $Lk^1$ est un lieur dégradable.

4. Conjugué selon la revendication 3, dans lequel $Lk^1$ comprend l'un des groupes suivantes :

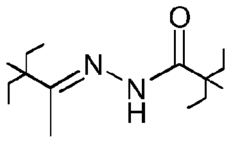
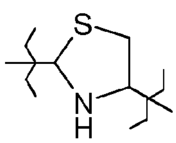
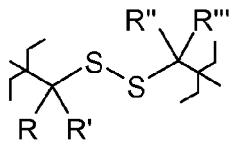
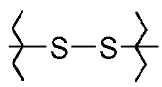

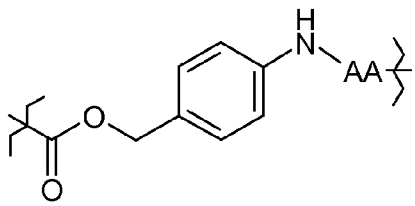
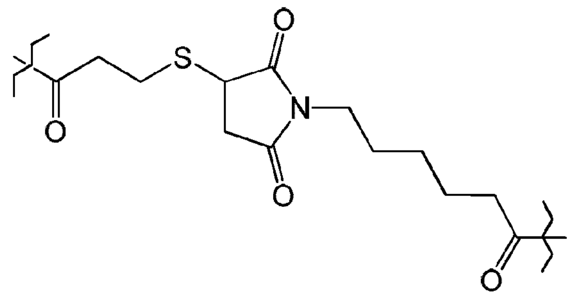

dans lesquels chacun de R, R', R" et R''' représente un atome d'hydrogène ou un groupe alkyle et AA représente une séquence d'acides aminés spécifique d'une protéase.

5. Conjugué selon la revendication 4, dans lequel $Lk^1$ comprend :

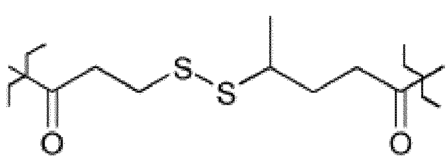
(Vb)
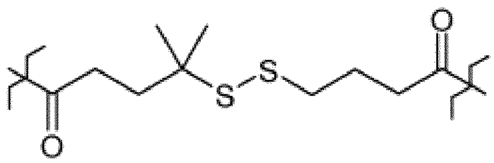
(Vc),

ou

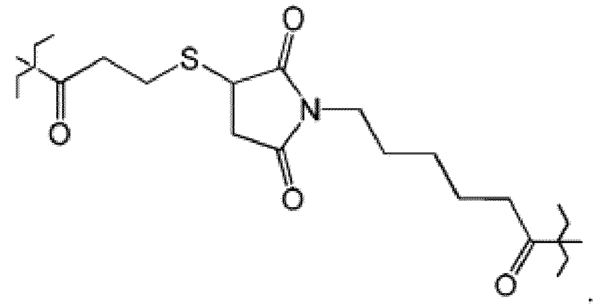

6. Conjugué selon la revendication 1 ou la revendication 2, dans lequel q est un nombre entier de 2 à 10 et $Lk^1$ est un lieur multivalent incorporant un ou plusieurs résidus aspartate ou glutamate.

7. Conjugué selon l'une quelconque des revendications 1 à 6, dans lequel P représente une liaison, ou P représente $-P^1-NH-$ dans lequel $P^1$ contient 2 à 10 unités éthylèneglycol.

8. Conjugué selon l'une quelconque des revendications 1 à 6, dans lequel $P^1$ représente le polyéthylèneglycol.

9. Conjugué selon l'une quelconque des revendications 1 à 8, dans lequel le groupe phényle Ph dans $Lk^3$ est non substitué.

10. Conjugué selon l'une quelconque des revendications 1 à 9, dans lequel Y a la formule :

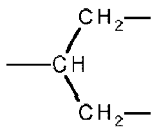

11. Conjugué selon l'une quelconque des revendications 1 à 10, dans lequel Ab représente un anticorps pleine longueur ou un fragment d'anticorps comprenant une région de liaison à un antigène de l'anticorps pleine longueur.

12. Conjugué selon la revendication 11, dans lequel Ab représente IgG1 ou IgG4 ou un fragment de IgG1 ou de IgG4.

13. Procédé de préparation d'un conjugué selon l'une quelconque des revendications 1 à 12, qui comprend la réduction d'une ou de plusieurs liaisons disulfure dans une protéine de liaison puis la réaction avec un réactif de conjugaison de formule générale :

$$((D_q-Lk^1)_m-P)_p-Lk^2-Lk^{3a} \qquad (VIII)$$

dans laquelle D, $Lk^1$, P, $Lk^2$ et m, p et q ont les significations données dans la revendication 1, et $Lk^{3a}$ représente un groupe de formule :

$$-CO-Ph-X^a-Y^a \qquad (IX)$$

dans laquelle Ph a la signification donnée dans la revendication 1, $X^a$ représente un groupe CO, et $Y^a$ représente un groupe :

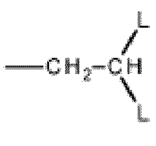 (X), 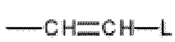 (XI), 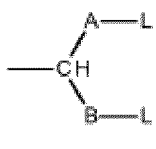 (XII)

ou

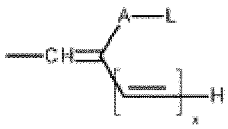 (XIII)

dans lesquelles A et B ont les significations données dans la revendication 1, chaque L représente indépendamment un groupe partant, et x représente un nombre entier de 1 à 4, pour produire un conjugué de formule I dans laquelle X représente CO ; et éventuellement la réduction dudit groupe X initialement formé CO pour donner un conjugué ayant un groupe X CH. OH.

**14.** Procédé selon la revendication 13, dans lequel $Y^a$ représente :

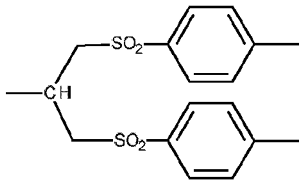 (XIIa) .

**15.** Composé de formule générale :

$$((D_q\text{-}Lk^1)_m\text{-}P)_p\text{-}Lk^2\text{-}Lk^{3a} \qquad (VIII)$$

dans laquelle D a la signification donnée dans l'une quelconque des revendications 1 à 3 ; $Lk^1$ a la signification donnée dans l'une quelconque des revendications 1 et 4 à 7 ; P a la signification donnée dans l'une quelconque des revendications 1, 8 et 9 ; $Lk^2$, m, p et q ont les significations données dans la revendication 1, et $Lk^{3a}$ représente un groupe de formule :

$$\text{-}CO\text{-}Ph\text{-}X^a\text{-}Y^a \qquad (IX)$$

dans laquelle Ph a la signification donnée dans la revendication 1 ou la revendication 10, $X^a$ représente un groupe CO, et $Y^a$ représente un groupe :

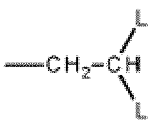 (X), 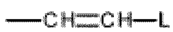 (XI), 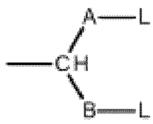 (XII)

ou

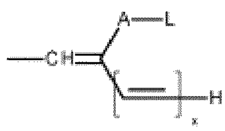 (XIII)

dans lesquelles A et B ont les significations données dans la revendication 1, chaque L représente indépendamment un groupe partant, et x représente un nombre entier de 1 à 4.

**16.** Composé selon la revendication 15, dans lequel $Y^a$ représente :

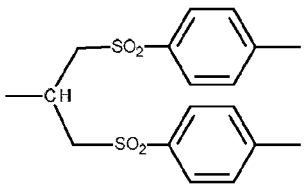

(XIIa) .

17. Composition pharmaceutique comprenant un conjugué selon l'une quelconque des revendications 1 à 12, conjointement avec un véhicule pharmaceutiquement acceptable, éventuellement conjointement avec un agent thérapeutique supplémentaire.

18. Conjugué selon l'une quelconque des revendications 1 à 12 ou composition selon la revendication 17 destiné à être utilisé en thérapie.

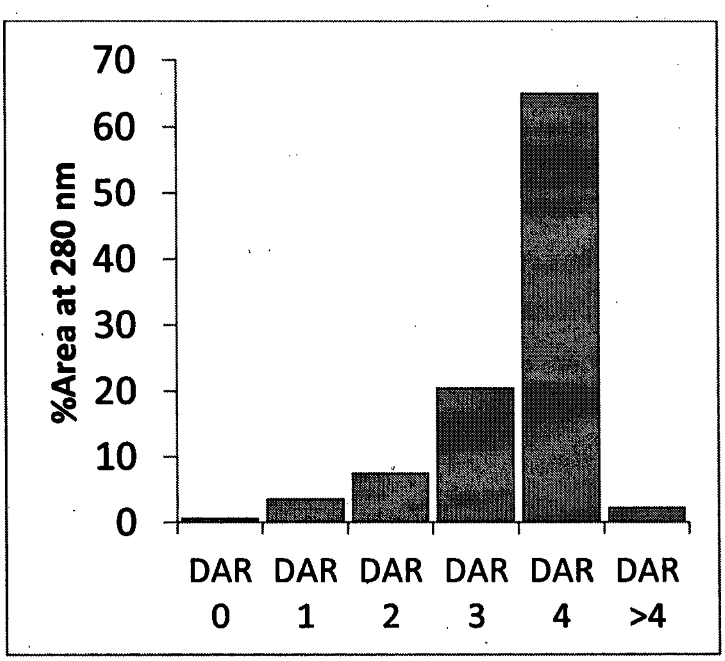


Figure 1

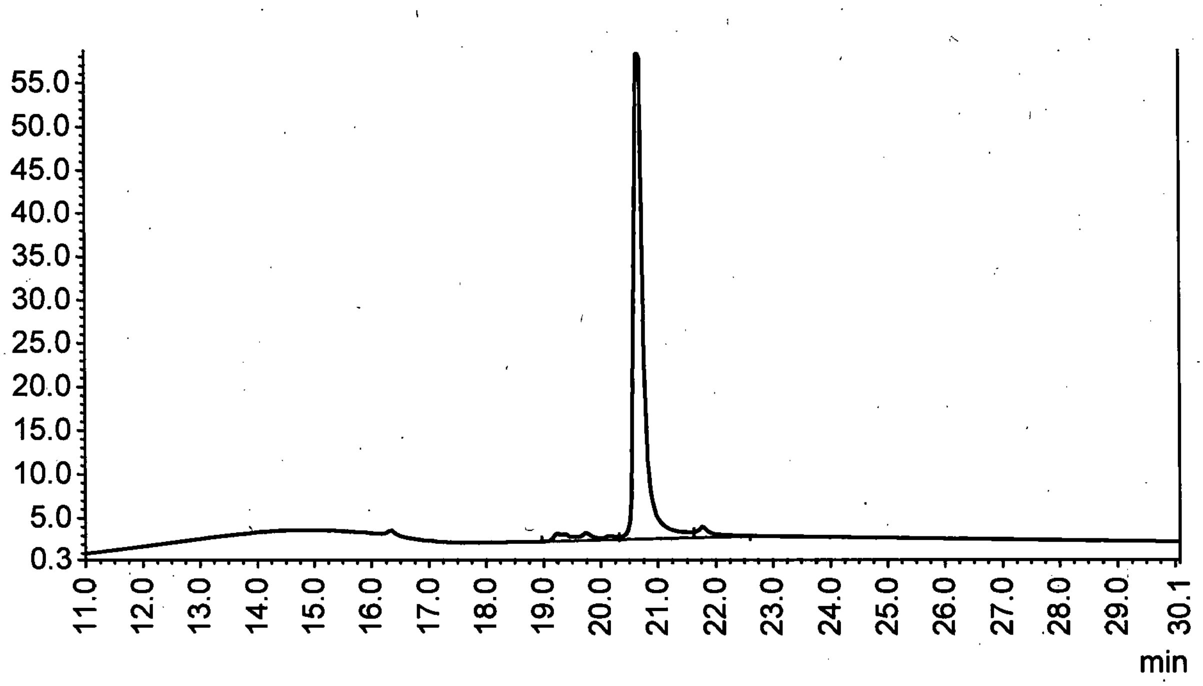


Figure 2

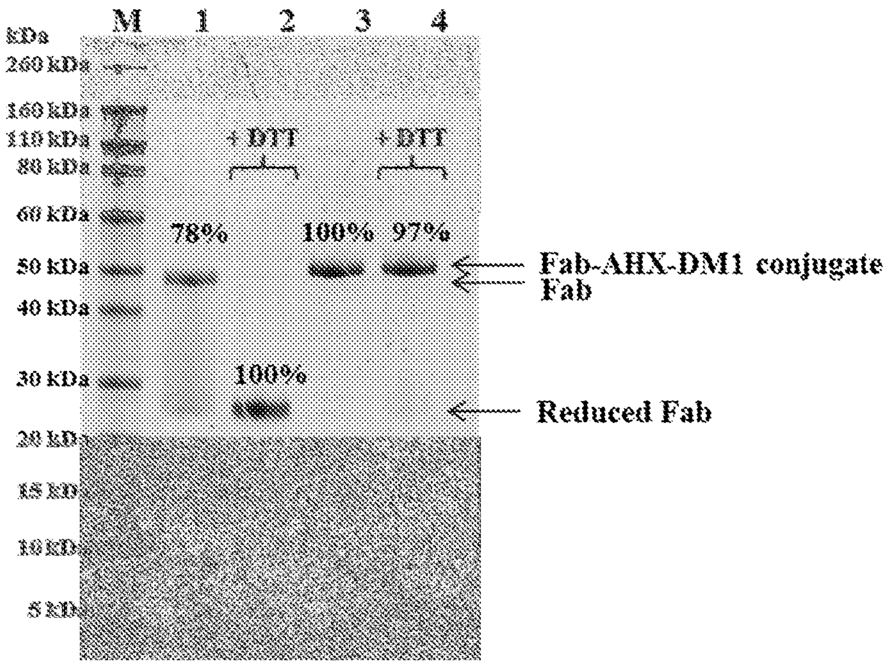
M
1
2
3
4
kDa
260 kDa
160 kDa
110 kDa
80 kDa
60 kDa
50 kDa
40 kDa
30 kDa
20 kDa
15 kD
10 kD
5 kD
+ DTT
+ DTT
78%
100%
97%
100%
Fab-AHX-DM1 conjugate
Fab
Reduced Fab

Figure 3

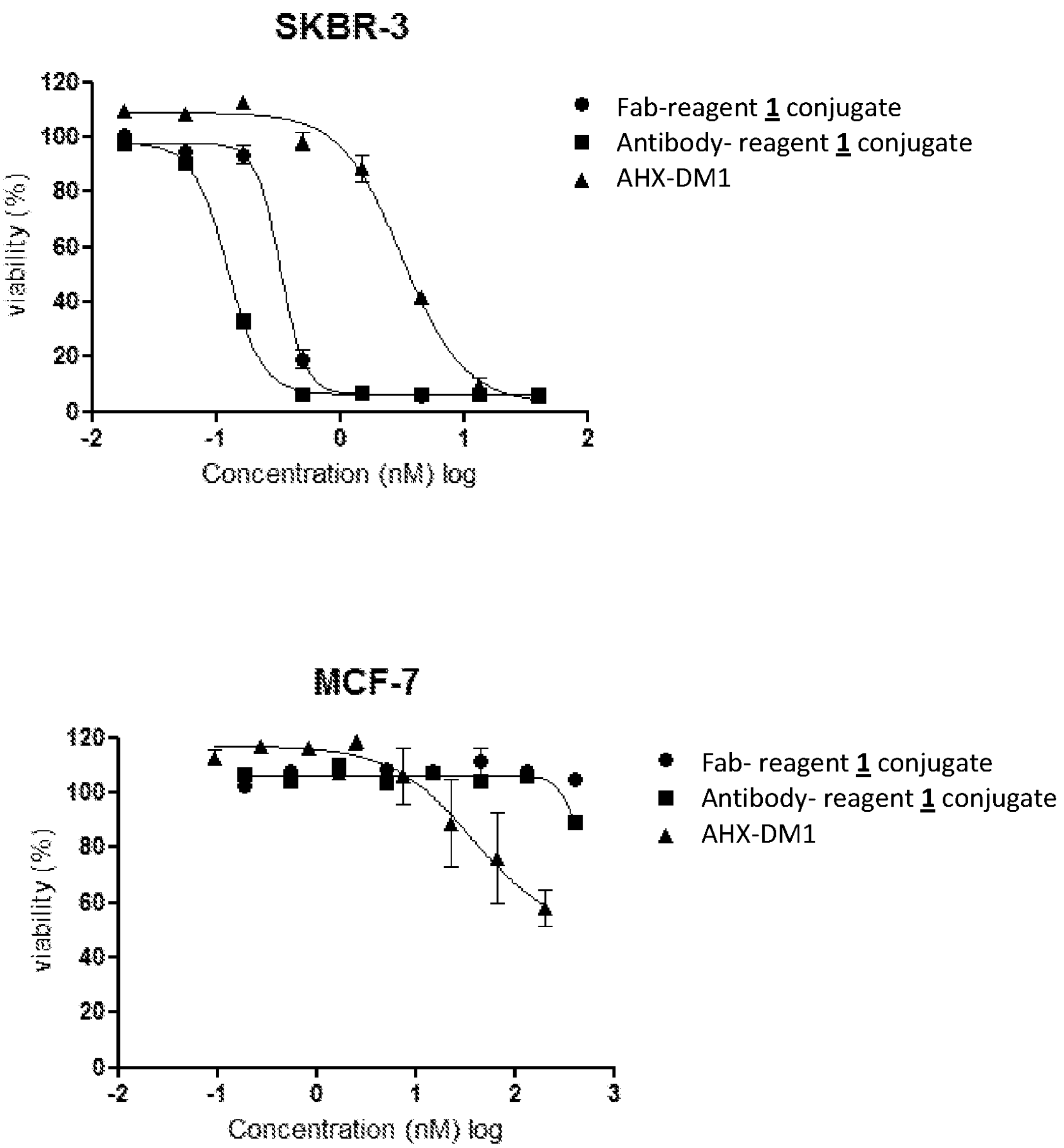
SKBR-3
viability (%)
120
100
80
60
40
20
0
-2
-1
0
1
2
Concentration (nM) log
Fab-reagent 1 conjugate
Antibody- reagent 1 conjugate
AHX-DM1
MCF-7
viability (%)
120
100
80
60
40
20
0
-2
-1
0
1
2
3
Concentration (nM) log
Fab- reagent 1 conjugate
Antibody- reagent 1 conjugate
AHX-DM1

**Figure 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005007197 A
- WO 2009134976 A
- WO 2011039721 A
- WO 2010100430 A


### Non-patent literature cited in the description


- **LIBERATORE et al.** *Bioconj. Chem,* 1990, vol. 1, 36-50
- **DEL ROSARIO et al.** *Bioconj. Chem.,* 1990, vol. 1, 51-59
- **KHALILI et al.** *Bioconjugate Chem.,* 2012, vol. 23, 2262-2277
- **CARTER P et al.** *Endocr. Relat. Cancer.,* December 2004, vol. 11 (4), 659-87
- **LIU et al.** *Anal. Chem.,* 2010, vol. 82, 5219-5226
- *Nat. Prot.,* 2006, vol. 1 (4
- **LEWIS PHILLIPS G.D.** *Cancer Res,* 2008, vol. 68, 9280-9290
- **R. IAN FRESHNEY.** Culture of Animal Cells: A Manual of Basic Technique. Alan R. Liss, 1994
- CULTURE OF ANIMAL CELLS: A MANUAL OF BASIC TECHNIQUE. Wiley-Liss, 2005
- *Nature Protocols,* 2006, vol. 1 (54), 2241-2252
- **WIDDISON et al.** *J Med. Chem.,* 2006, vol. 49 (14), 4392-4408